# FASCICULE DE BREVET EUROPEEN

(11) **EP 1 611 186 B1**
(45) Date de publication et mention de la délivrance du brevet: **12.10.2011**
(21) Numéro de dépôt: 04742832.1
(22) Date de dépôt: 06.04.2004
(51) Int. Cl.: C08G 73/10, C07C 65/00, C07C 63/00, C07C 51/00

(54) **POLYIMIDES PERDEUTERES, LEUR PROCEDE DE PREPARATION ET LEUR UTILISATION EN TANT QUE MATERIAUX TRANSPARENTS DANS LA ZONE DE 2500 A 3500 CM-1**
PERDEUTERIERTE POLYIMIDE, PROZESS ZU IHRER HERSTELLUNG UND IHRE VERWENDUNG ALS TRANSPARENTES MATERIAL IM BEREICH VON 2500 BIS 3500 CM-1
PERDEUTERATED POLIIMIDES, METHOD FOR THE PRODUCTION AND USE THEREOF IN THE FORM OF TRANSPARENT MATERIALS IN THE RANGE OF 2500-3500 CM-1

(30) Priorité: 08.04.2003 FR 0350090
(43) Date de publication de la demande: 04.01.2006
(73) Titulaire: Commissariat à l'Énergie Atomique et aux Énergies Alternatives, 75015 Paris (FR)
(72) Inventeur: ANSELMI, Elsa, F-92380 Garches (FR); RABY, Jacques, F-38000 Grenoble (FR); BALLAND-LONGEAU, Alexia, F-37000 Tours (FR); CALONNE, Marc, F-37800 DRACHE (FR)
(74) Mandataire: Poulin, Gérard
(86) Numéro de dépôt international: PCT/FR2004/050145
(87) Numéro de publication internationale: WO 2004/092249

(56) Documents cités:
- US-A- 6 048 968
- WALLACE W E ET AL: "Gas absorption during ion-irradiation of a polymer target" 1 décembre 1995 (1995-12-01), NUCLEAR INSTRUMENTS & METHODS IN PHYSICS RESEARCH, SECTION - B: BEAM INTERACTIONS WITH MATERIALS AND ATOMS, NORTH-HOLLAND PUBLISHING COMPANY. AMSTERDAM, NL, PAGE(S) 435-439 , XP004000518 ISSN: 0168-583X pages 435-437

## Description

### DOMAINE TECHNIQUE

L'invention a trait à des polyimides aromatiques deutérés, présentant à la fois d'excellentes propriétés mécaniques, thermiques et optiques et présentant une transparence dans la zone de 2500 à 3500 cm⁻¹ du spectre infrarouge et à un procédé de préparation de ces polymères et la mise en oeuvre de ces polymères sous forme de films.

Ces polyimides trouvent notamment leur application en raison de leurs excellentes propriétés mécaniques, thermiques, et optiques dans la préparation de matériaux organiques pour lasers de puissance utilisés notamment lors de la réalisation d'expériences de physique nucléaire.

Le domaine général de l'invention est donc celui des matériaux organiques, présentant une transparence dans une gamme de longueurs d'onde donnée.

On précise que, dans le cadre de cette description, on entend par matériaux transparents dans une gamme de longueur d'onde donnée, des matériaux aptes à laisser passer, sans absorption, des signaux optiques de longueur(s) d'onde appartenant à la gamme susmentionnée.

D'une façon générale, les matériaux organiques, tels que les polymères organiques, peuvent engendrer une certaine atténuation optique des signaux optiques les traversant, c'est-à-dire une perte d'intensité de ces signaux lumineux. Cette atténuation optique observée avec les polymères organiques peut être attribuée à l'absorption de certaines longueurs d'onde par les liaisons constitutives du polymère (telle que l'absorption des harmoniques des bandes de vibration de valence des liaisons C-H) et également à la diffusion. Cette atténuation optique est ainsi liée directement à la structure chimique du polymère.

### ETAT DE LA TECHNIQUE

De nombreux travaux ont ainsi porté sur la recherche de polymères organiques présentant une structure chimique apte à diminuer les pertes optiques liées à l'absorption.

Ainsi, l'auteur Kaino dans l'article 'Polymers for Optical Transmission and Optical Signal Processing', Reports on Progress in Polymer Physics in Japan, vol 43, 2000 [1] décrit des polymères deutérés et/ou fluorés tels que le polyméthacrylate de méthyle (PMMA) et le polystyrène (PS) présentant une atténuation optique minorée par rapport à leurs analogues non deutérés et/ou fluorés. Toutefois, ces polymères présentent l'inconvénient d'être peu stables thermiquement, dans la mesure où ils ne peuvent être utilisés pour des températures allant au-delà de 80°C. Ces polymères ne peuvent ainsi être utilisés dans des domaines tels que l'optoélectronique et les lasers à haute puissance, qui nécessitent des propriétés thermiques bien supérieures à celles de ces polymères.

Des polymères susceptibles de présenter de meilleures propriétés mécaniques et thermiques sont les polyimides aromatiques. Toutefois, ces polymères présentent, du fait de la présence d'un grand nombre de liaisons C-H, des pertes optiques très importantes. Afin de contrer cet inconvénient, de nombreux auteurs ont cherché à modifier la structure de ces polymimides, notamment en modifiant les liaisons C-H de façon à obtenir des polyimides présentant des pertes optiques les plus faibles possibles, en particulier dans le domaine de l'infra-rouge.

L'auteur du document [1] et les auteurs Saint-Clair et al. dans l'article « Evaluation of Colorless Polyimide Film for Thermal Control Coating Applications », Sampe Journal, August 1985, pp 28-33 [2], ont décrit un polyimide aromatique comprenant um motif :

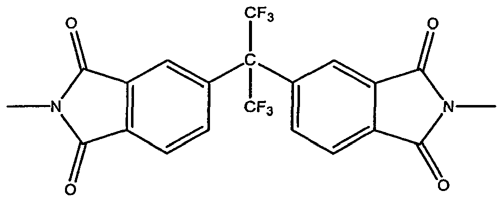

Du fait de la présence d'un groupement hexafluoroisopropylidène, ces polyimides présentent une absorption plus faible dans l'infra-rouge que les analogues hydrogénés et donc une perte optique plus faible dans cette zone. Toutefois, ces polymères comprennent encore des liaisons C-H dans le groupement phényle et donc une absorption importante dans la zone de 2500 à 3500 cm⁻¹ du spectre infrarouge. Ceci écarte ces polyimides d'une utilisation pour une application dans le domaine des lasers à haute puissance.

Les auteurs Ando et col. dans les brevets américains US 5,233,018 [3] et US 6,048,986 [4] décrivent des polyimides perfluorés de manière à obtenir une diminution des pics d'absorption dans une fenêtre de transmission optique située dans le proche infra-rouge, c'est-à-dire de 5880 à 10 000 cm⁻¹. Toutefois, ces polymères ne sont pas totalement transparents dans le domaine situé de 2500 à 3500 cm⁻¹, à savoir un domaine de transmission mise en oeuvre dans les lasers à haute puissance.

Enfin, les auteurs Wallace et al. dans l'article 'Gas Absorption during ion-irradiation of a polymer target', Nuclear Instruments and Methods in Physics Research B 103 (1995),435-439 [5] ont décrit un polyimide partiellement deutéré (à 23%) comprenant un motif dianhydride pyromellitique-oxydianiline. Toutefois, ce polymère présente des propriétés mécaniques, telle qu'une contrainte à la rupture d'environ 110 Mpa, et des propriétés optiques insuffisantes pour une application dans le domaine des lasers à haute puissance.

Ainsi, les polymères de l'art antérieur présentent tous l'un ou plusieurs des inconvénients suivants :
- ils présentent des propriétés thermiques insuffisantes ;
- ils présentent des pics d'absorption d'intensité trop importante (c'est-à-dire une atténuation optique trop importante) dans une fenêtre de transmission donnée, en particulier dans la fenêtre de transmission comprise entre 2500 cm⁻¹ à 3500 cm⁻¹ ;
- ils présentent des propriétés mécaniques difficilement compatibles avec la mise en oeuvre de ces polymères dans des domaines nécessitant des propriétés mécaniques très élevées.

### EXPOSÉ DE L'INVENTION

Un but de la présente invention est de proposer de nouveaux polymères ne présentant pas les inconvénients susmentionnés des polymères de l'art antérieur, et présentant notamment de hautes performances mécaniques (telle qu'une contrainte à la rupture supérieure à 110 MPa) et une totale transparence dans une zone de transmission de 2500 à 3500 cm⁻¹_{.}

Un but de la présente invention est également de proposer un procédé de préparation de polymères conformes à la présente invention.

Un autre but de la présente invention est de proposer des monomères utilisables dans le cadre du procédé de l'invention.

Un but de la présente invention est également de proposer des procédés de préparation de tels monomères.

Enfin, un but de la présente invention est de proposer des films à base de polymères conformes à l'invention.

La présente invention a trait, selon un premier objet, à un polyimide deutéré, dont le squelette comprend une alternance entre:
- au moins un motif répétitif répondant à la formule (I) suivante : 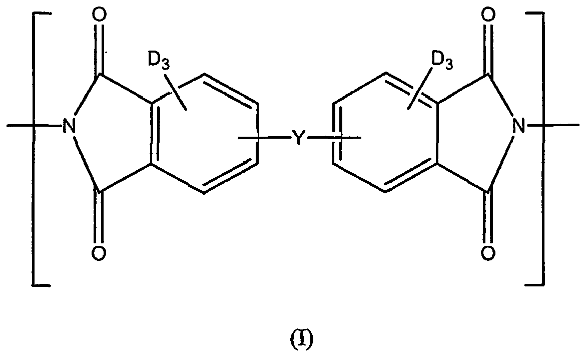 dans laquelle :
- Y représente une liaison simple ou un groupe espaceur ; et
- au moins un motif répétitif répondant à la formule (II) suivante : 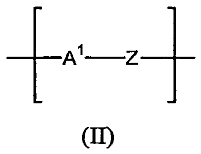 dans laquelle :
- A¹ représente un groupe aromatique perdeutéré comportant de 6 à 10 atomes de carbone ;
- Z représente une liaison simple ou un groupe choisi parmi -O-C₆D₄-, -CO-C₆D₄-, -C₆D₄-.

Ainsi, les polyimides de l'invention correspondent à des polymères alternés, dont le squelette comprend une alternance entre au moins un motif de formule (I) et au moins un motif de formule (II). En d'autres termes, lesdits motifs s'enchaînent de la manière suivante :

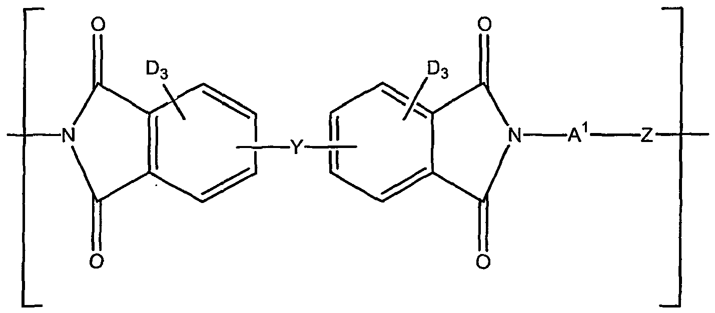

Outre l'alternance entre au moins un motif de formule (I) et au moins un motif de formule (II), le squelette des polyimides de l'invention peut comprendre d'autres motifs tels qu'un motif de formule (III), telle qu'explicitée ci-dessous.

Lorsque les polyimides de l'invention comprennent différents motifs répétitifs de formule (I) et différents motifs répétitifs de formule (II), l'alternance dans le squelette entre les différents motifs de formule (I) et les différents motifs de formule (II) sera aléatoire.

On entend, selon l'invention, par liaison simple, une liaison covalente. Ainsi, lorsque Z représente une liaison simple, le motif de formule (II) correspond à un motif de formule -A¹-.

On précise que par groupe espaceur on entend un groupe formant pont entre les deux groupes phényles et lié par liaison covalente à ceux-ci.

On précise que D₃ signifie que les cycles phényle sont substitués par 3 atomes de deutérium.

De manière surprenante, les auteurs de cette invention ont pu déterminer que ces polyimides présentent d'excellentes propriétés mécaniques, telles qu'une contrainte à la rupture σᵣ supérieure à 110 Mpa, un module d'Young E supérieure à 2 Gpa et une déformation à la rupture εᵣ supérieure ou égale à 10 %.

De plus, les polyimides de l'invention sont aptes à supporter des températures comprises entre -253 et 400°C, ce qui rend ces polyimides applicables dans une très large gamme de températures.

Enfin, le fait que ces polyimides comportent des groupes aromatiques perdeutérés contribue à rendre ces polymères transparents dans le domaine de l'infra-rouge compris entre 2500 et 3500 cm⁻¹.

Comme mentionné précédemment, les polyimides de l'invention correspondent à des polyimides aromatiques perdeutérés, c'est-à-dire dont tous les atomes d'hydrogène portés par les groupes aromatiques sont remplacés par des atomes de deutérium.

On précise que par groupe aromatique perdeutéré, on entend, dans ce qui précède et ce qui suit, un groupe benzénique perdeutéré ou un groupe naphtalénique perdeutéré.

Le groupe Y formant pont entre les groupes phényls peut être une liaison simple ou un groupe espaceur. Lorsque Y est un groupe espaceur, il peut être choisi parmi -O-, -CD₂-, -CO-, -SO₂-, -C₆D₄-.

De préférence, le nombre de répétition des motifs de formule (I) est égal au nombre de répétition des motifs de formule (II).

Des polyimides particuliers conformes à la présente invention sont des polyimides, dont le squelette comprend une alternance entre un motif répétitif de formule (I) définie ci-dessus et un motif répétitif de formule (IIa) : 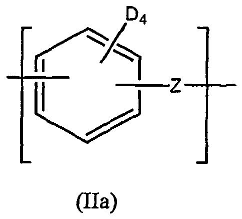 dans laquelle Z répond à la même définition que celle donnée précédemment.

Des polyimides encore plus particuliers sont les polyimides choisis parmi :
- les polyimides comprenant un motif répétitif de formule (Ia) et un motif répétitif de formule (IIb) suivantes : 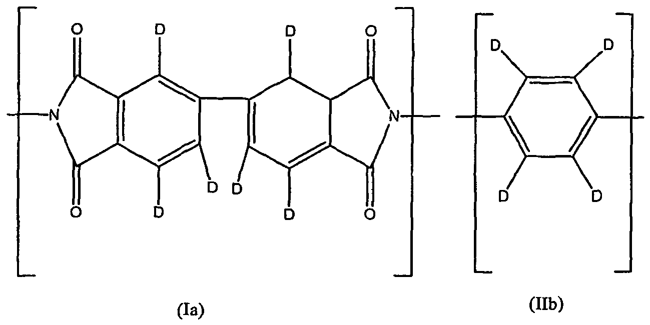
- les polyimides comprenant un motif répétitif de formule (Ia) et un motif répétitif de formule (IIc) suivantes : 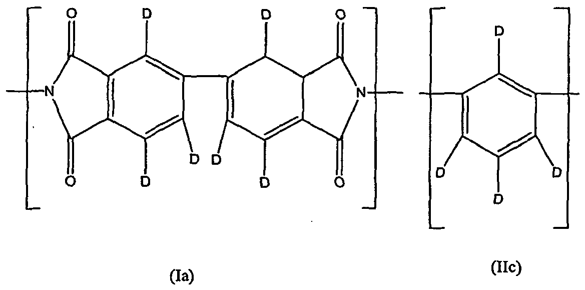
- les polyimides comprenant un motif répétitif de formule (Ia) et un motif répétitif de formule (IId) suivantes : 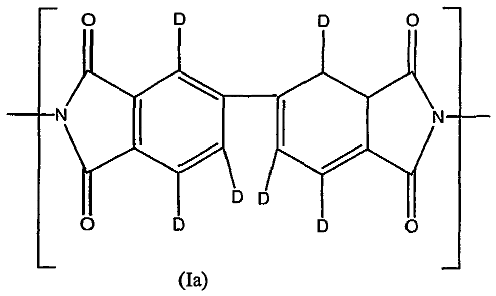 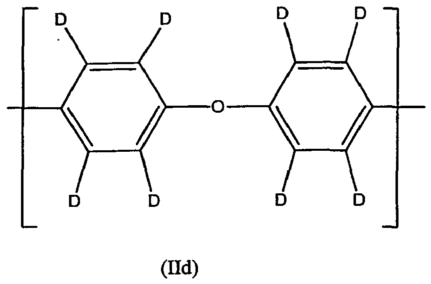
- les polyimides comprenant un motif répétitif de formule (Ib) et un motif répétitif de formule (IIb) suivantes : 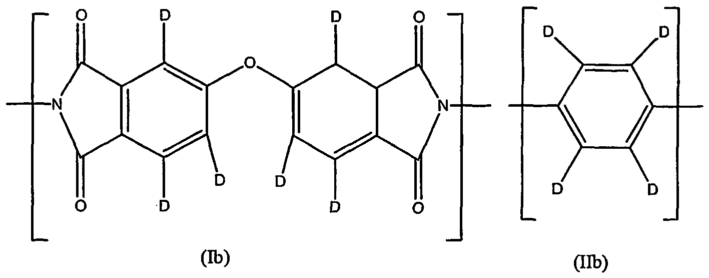
- les polyimides comprenant un motif répétitif de formule (Ib) et un motif répétitif de formule (IId) suivantes : 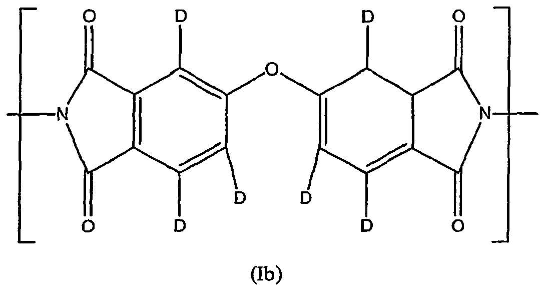 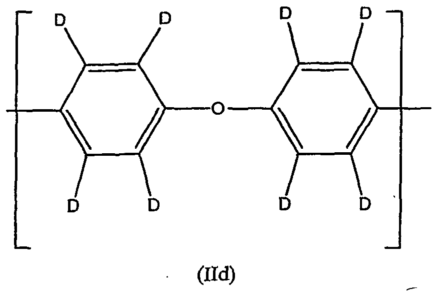
- les polyimides comprenant un motif répétitif de formule (Ic) et un motif répétitif de formule (IIb) suivantes : 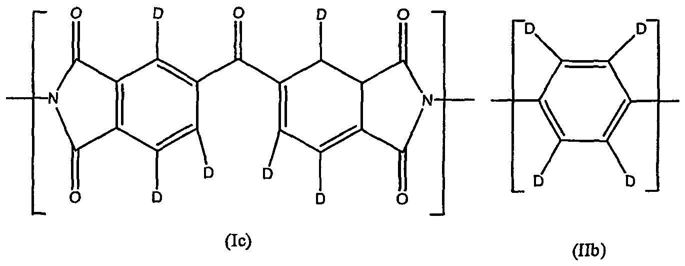
- les polyimides comprenant un motif répétitif de formule (Ic) et un motif répétitif de formule (IId) suivantes : 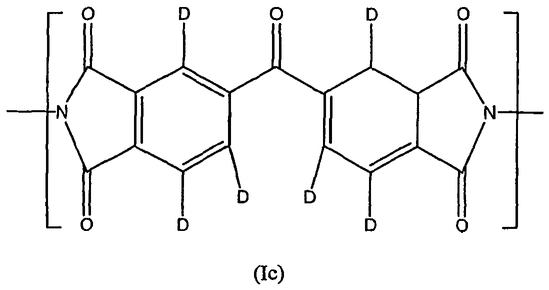 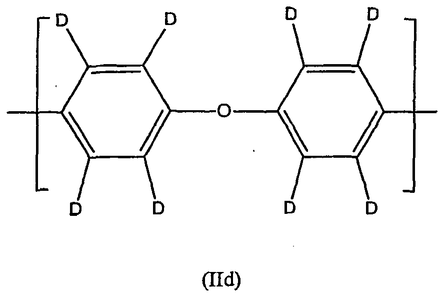
- les polyimides comprenant un motif répétitif de formule (Id) et un motif répétitif de formule (IId) suivantes : 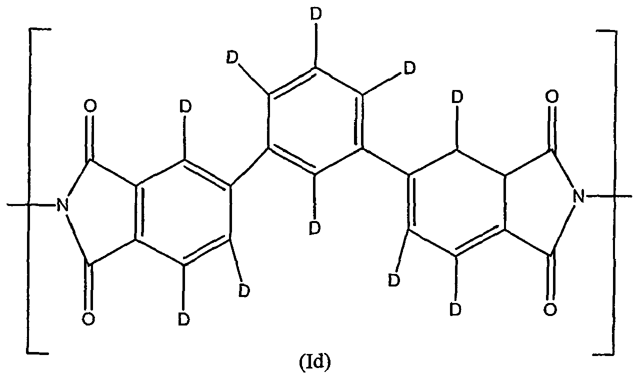 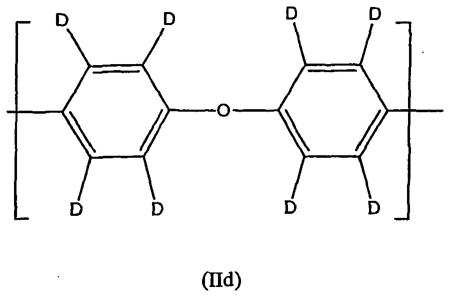
- les polyimides comprenant un motif répétitif de formule (Ia), un motif répétitif de formule (IIb) et un motif répétitif de formule (IId) suivantes : 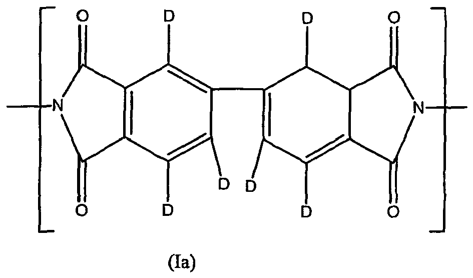 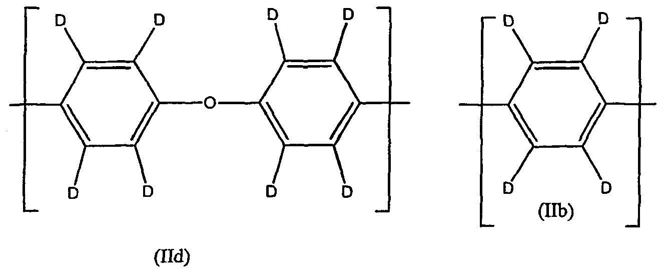
- les polyimides comprenant un motif répétitif de formule (Ic), un motif répétitif de formule (IIb) et un motif répétitif de formule (IId) suivantes :

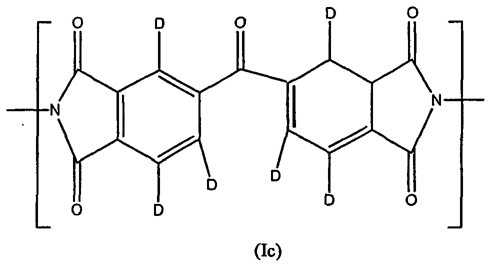

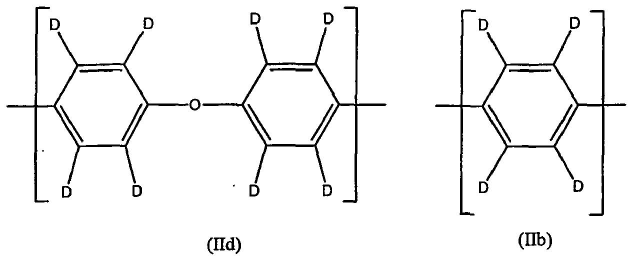

Il est entendu, que selon l'invention, ces polyimides particuliers comprennent un squelette constitué d'une alternance entre un motif particulier rentrant dans la définition des motifs de formules (I) et un motif particulier rentrant dans la définition des motifs de formule (II).

Ces polymères particuliers présentent notamment une totale transparence dans la zone s'échelonnant de 2500 à 3500 cm⁻¹ et d'excellentes propriétés mécaniques, telles qu'une contrainte à la rupture supérieure à 110 Mpa.

Selon l'invention, les polyimides deutérés peuvent comprendre, en outre, d'autres motifs imides, en particulier, un motif imide répondant à la formule (III) suivante :

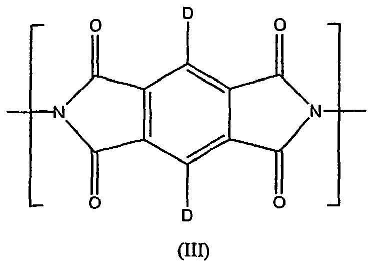

Dans ce cas de figure, les polyimides comporteront un squelette formé d'une alternance entre un motif de formule (I) et un motif de formule (II) et d'une alternance entre un motif de formule (II) et le motif de formule (III).

En d'autres termes, le squelette comportera les motifs suivants : 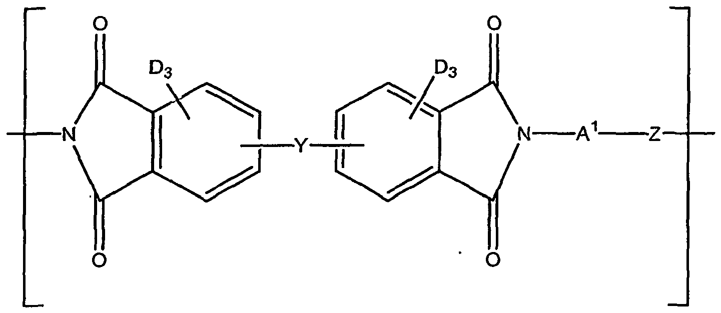 et : 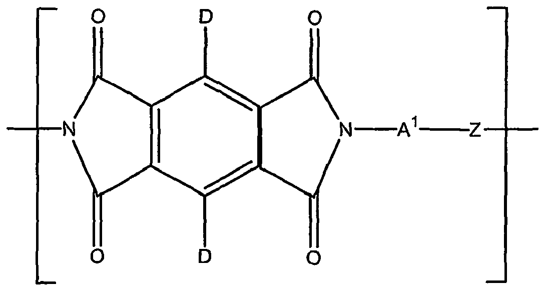 l'ordre d'enchaînements de ces motifs étant aléatoire.

Des polyimides particuliers répondant à la définition donnée dans le paragraphe précédent sont des polyimides comprenant un motif répétitif de formule (Ia), un motif répétitif de formule (IIb) et un motif répétitif de formule (III) suivantes :

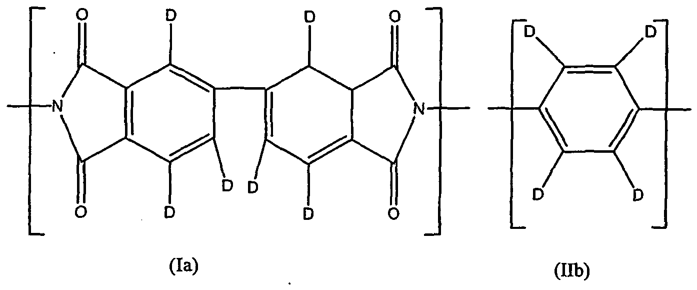

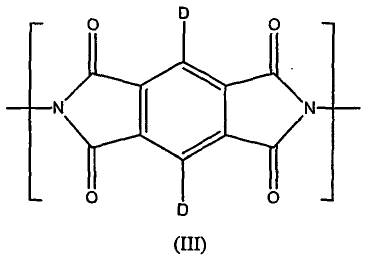

Dans ce cas, l'alternance dans le squelette se fera entre le motif de formule (Ia) et (IIb) et entre le motif de formule (III) et le motif de formule (IIb), ceci de manière aléatoire.

Les polyimides conformes à la présente invention peuvent être préparés par tout type de procédé.

En particulier, les polyimides de l'invention peuvent être préparés par un procédé, comprenant une étape consistant à traiter par un chauffage à une température adéquate une solution d'un poly(amide-acide), dont le squelette comprend une alternance entre au moins un motif répétitif de formule (IV) : 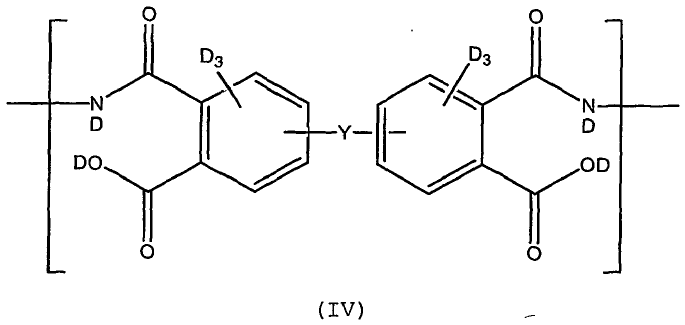 dans laquelle Y répond à la même définition que celle donnée précédemment ; et
au moins un motif répétitif de formule (II) : 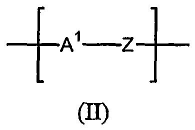 dans laquelle A¹ et Z répondent aux mêmes définitions que celles données précédemment, la température de chauffage adéquate étant déterminée de manière à obtenir une imidisation totale dudit poly(amide-acide).

Lorsque les polyimides de l'invention comprennent également un motif de formule (III) tel que défini ci-dessus, ces polyimides sont préparés à partir d'une solution de poly(amide-acide), dont le squelette comprend à la fois :
- une alternance entre au moins un motif répétitif de formule (IV) et un motif de formule (II), ce qui revient à dire que le poly(amide-acide) comprend un motif répétitif de formule : 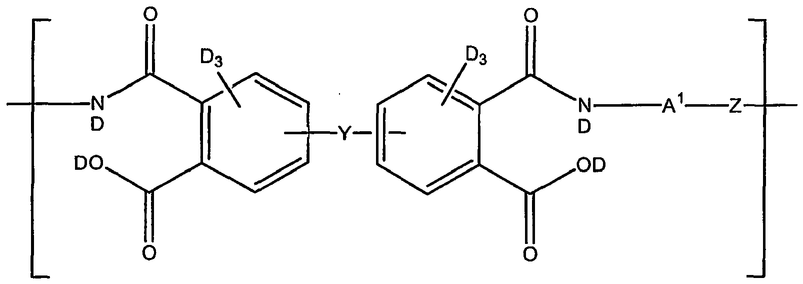 et
- une alternance entre un motif de formule (IVa) : 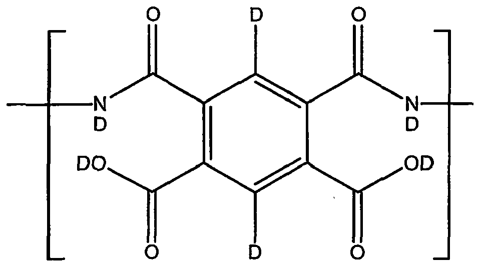 et au moins un motif de formule (II), ce qui revient à dire que le poly(amide-acide) comprend un motif répétitif de formule suivante :

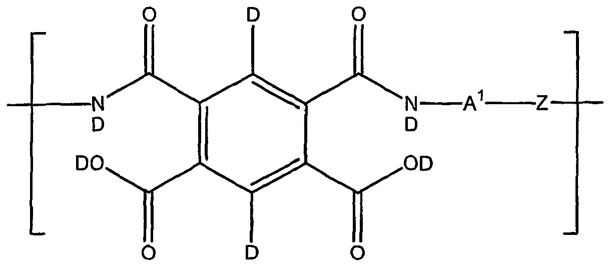

Le chauffage à une température adéquate peut s'effectuer à l'air, ou de préférence sous atmosphère de gaz inerte, tel qu'une atmosphère d'argon ou d'azote, à une température s'échelonnant, par exemple, de 80 à 400 °C pendant une durée s'échelonnant, par exemple, de 1 à 8 heures.

Selon le procédé de l'invention, la solution de poly(amide-acide) susmentionné peut être préparée par polycondensation dans un solvant d'au moins un monomère de formule (V) suivante : 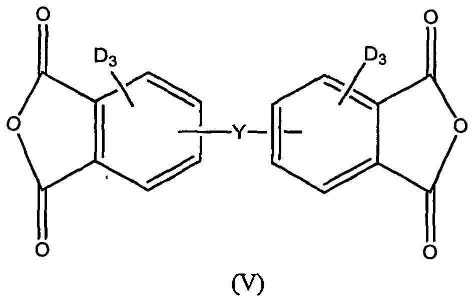 dans laquelle Y répond à la même définition que celle donnée précédemment, et d'au moins un monomère de formule (VI) suivante:

ND₂-A¹-Z-ND₂ (VI)

dans laquelle A¹ et Z répondent aux mêmes définitions que celles données précédemment, les monomères de formules (V) et (VI) étant, de préférence, mis à réagir en proportions stoechiométriques.

Lorsque la solution de poly(amide-acide) comprend également un motif de formule (IVa), la polycondensation se fera en présence également d'un monomère de formule suivante :

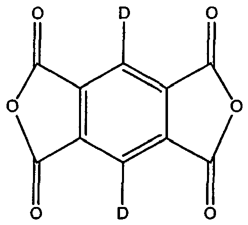

La préparation de cette solution de poly(amide-acide) est effectuée, de préférence, dans un solvant dipolaire aprotique, tel que du N-méthylpyrrolidone (NMP), du diméthylformamide (DMF) et du diméthylacétamide (DMAC), à température ambiante sous atmosphère de gaz inerte, la concentration de la solution pouvant varier, par exemple, de 5 à 15%.

Pour obtenir un précurseur poly(amide-acide) de masse moléculaire élevée, les monomères susmentionnés doivent être, de préférence, mis en présence, en proportions stoechiométriques. De plus, les monomères seront avantageusement purifiés avant utilisation, pour éliminer toute trace d'eau, qui pourrait engendrer l'hydrolyse du monomère dianhydride et toute trace d'impuretés qui pourrait déséquilibrer la stoechiométrie. Ces monomères peuvent être purifiés, par exemple, par sublimation.

L'invention a également pour objet des monomères dianhydrides pouvant être utilisés dans le cadre du procédé de l'invention, répondant à la formule (V) suivante : 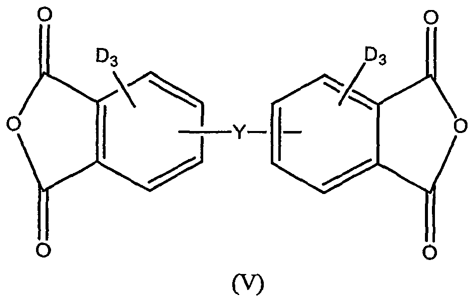 dans laquelle Y répond à la même définition que celle donnée précédemment.

Des monomères particuliers conformes à la formule (V) sont des monomères de formules suivantes :

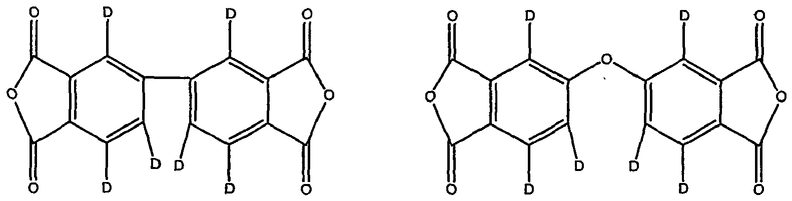

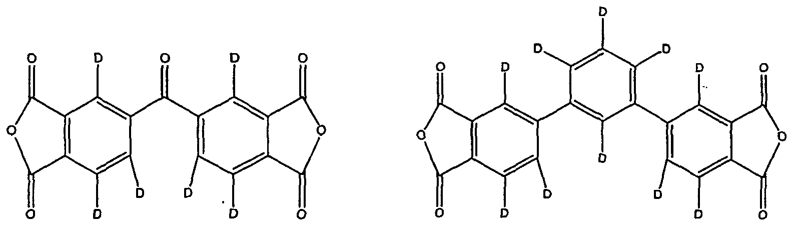

Les monomères diamines deutérés, pouvant être utilisés dans le cadre du procédé de l'invention, sont des monomères répondant à la formule générale (VI) suivante :

ND₂-A¹-Z-ND₂ (VI)

dans laquelle A¹ et Z répondent aux mêmes définitions que celles données précédemment.

Des monomères diamines particuliers conformes à la formule (VI) sont les monomères de formules suivantes :

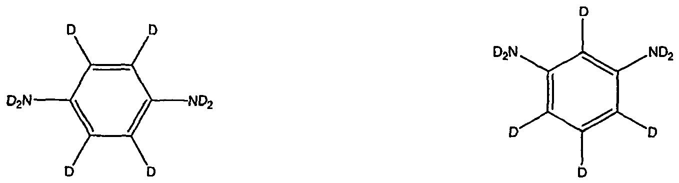

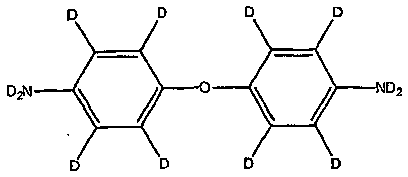

Ces monomères diamines deutérés sont notamment disponibles chez Aldrich et CDN-Isotopes.

L'invention a également pour objet un procédé de préparation de monomères de formule (V) suivante : 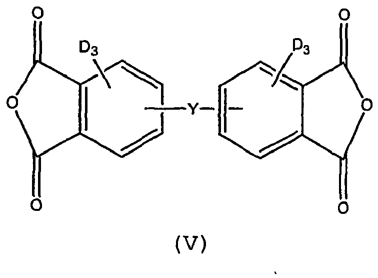 dans laquelle Y répond à la même définition que celle donnée précédemment, ledit procédé comprenant successivement les étapes suivantes :
- soumettre un composé de formule (VII) suivante : 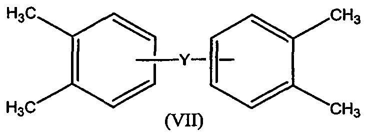 à une deutération de façon à obtenir un composé de formule (VIII) suivante: 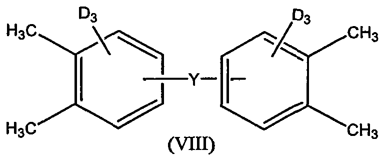
- soumettre le composé précédemment obtenu à une oxydation, de manière à obtenir un composé de formule (IX) suivante : 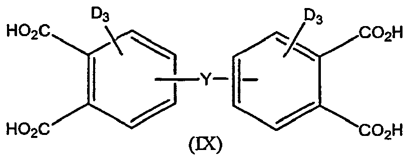
- soumettre le composé précédemment obtenu à une cyclodéshydratation, de façon à obtenir le composé de formule (V).

Les composés tétraméthylés de départ, représentés par la formule (VII) sont des composés disponibles commercialement chez Lancaster ou peuvent être préparés, le cas échéant, par des méthodes de synthèse à la portée de l'homme du métier. Ces composés de départ, conformément au procédé de l'invention, subissent une étape de deutération totale des noyaux aromatiques sans deutération des groupes méthyles, de façon à obtenir les composés représentés par la formule (VIII). Cette étape de deutération est effectuée avantageusement par chauffage à une température adéquate, par exemple de 250°C, du composé tétraméthylé de départ en milieu acide deutéré (tel qu'une solution de DCl) et en présence d'eau deutérée, sous une pression moyenne, c'est-à-dire une pression pouvant s'échelonner jusqu'à 40-45 bars. Cette étape de deutération est avantageusement mise en oeuvre dans un appareil de Parr, qui est un appareil destiné aux réactions à réaliser sous pression moyenne. L'étape de deutération explicitée ci-dessus est décrite, plus en détail, dans la publication de Werstiuk et al. « The High Temperature and Dilute Acid (HTDA) Procedure as a General Method of Replacing Aromatic Hydrogen by Deuterium », Can.J.Chem, vol 52, 2169-2171, 1973 [6].

Les composés deutérés de formule (VIII) subissent ensuite une étape d'oxydation destinée à transformer les groupes méthyles en groupes -COOH. Avantageusement, cette étape d'oxydation est mise en oeuvre en faisant réagir le composé de formule (VIII) avec du permanganate de potassium en milieu biphasique (phase aqueuse/phase organique) en présence d'un agent de transfert de phase. La phase organique peut se composer, par exemple, d'un solvant halogéné tel que le dichloroéthane et l'agent de transfert de phase peut être un sel d'ammonium, tel que le bromure de tétrabutylammonium ou le bromure de cétyltriméthylammonium. De plus amples renseignements concernant cette étape d'oxydation, et notamment le mécanisme réactionnel mis en jeu, figurent dans la publication de Artamkina et al. dans « Oxidation of Alkyl Aromatic Compounds With Potassium Permanganate Under The Conditions of Interphase Catalysis », traduit de Zhurnal Organicheskoi Khimii, vol.16, n°4, 99, 698-702, April 1980 [7].

Enfin, le composé tétracarboxylique de formule (IX) est soumis à une étape de cyclodéshydratation. Cette étape peut être effectuée soit par sublimation du composé (IX), soit par chauffage en présence d'anhydride acétique, étape à l'issue de laquelle, l'on obtient les monomères dianhydrides perdeutérés souhaités.

Alternativement, le procédé de préparation d'un monomère de formule (V) peut consister à soumettre un composé de formule (X) : 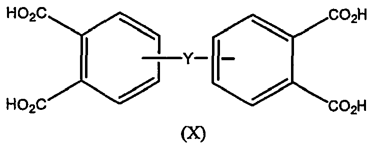 à une deutération des noyaux aromatiques, à une pression supercritique, de façon à obtenir le composé de formule (V) susmentionné.

On précise, que la pression supercritique correspond à une pression sensiblement égale à 220 bars. Les composés de formule (X) peuvent être disponibles commercialement chez Interchim ou préparés par des méthodes de synthèse classiques à la portée de l'homme du métier. L'étape de deutération est similaire à celle déjà décrite précédemment.

L'invention a trait également à un procédé de préparation de monomères de formule (VI) : 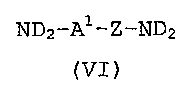 dans laquelle A¹ et Z répondent aux mêmes définitions que celles données précédemment, ledit procédé comprenant successivement les étapes suivantes :
- faire réagir un composé de formule (XI) suivante: 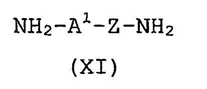 avec un acide minéral HX de façon à obtenir un sel d'ammonium de formule (XII) suivante : 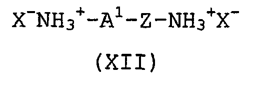 dans laquelle X représente un halogénure, tel qu'un chlorure ou un bromure ;
- faire réagir ledit sel d'ammonium avec de l'eau deutérée sous une pression adéquate, suivie d'une réaction avec une base de manière à obtenir le monomère de formule (VI).

Alternativement, le procédé de préparation d'un monomère de formule (VI) peut consister à faire réagir le composé de formule (XI) suivante: 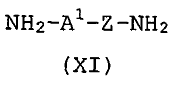 avec de l'eau deutérée en milieu basique sous une pression adéquate pour obtenir un monomère de formule (VI) .

Les composés de base de formule (XI) sont des composés disponibles commercialement chez Aldrich et Interchim. La préparation d'un sel d'ammonium consiste à faire réagir sur le composé diamine de formule (XI) un acide tel que l'acide chlorhydrique. Le sel d'ammonium formé subit ensuite une étape de deutération consistant à échanger les hydrogènes portés par le ou les groupe (s) aromatique (s) et les groupes amines par du deutérium par action de l'eau deutérée suivi d'une étape finale de traitement avec une base telle que NaOH ou NaOD pour obtenir les monomères diamines deutérés souhaités. De préférence, l'étape de deutération est mise en oeuvre à une température allant de 100 à 375°C sous une pression moyenne, par exemple de 15 à 50 bars voire jusqu'à 220 bars, de préférence, dans un appareil de Parr.

L'invention a trait à un film (ou membrane) à base d'un polyimide deutéré tel que défini précédemment.

On précise que, selon l'invention, on entend par film (ou membrane) une couche uniforme de polyimide sur un support, cette couche résultant d'un dépôt sur ledit support d'une solution de (polyamide-acide) définie précédemment ladite solution ayant subi un traitement d'imidisation totale. On précise que cette couche peut être maintenue sur le support (« film supporté) ou peut être détachée de ce même support (« film autosupporté »).

Ce film peut être préparé par tout type de procédés connus de l'homme du métier.

En particulier, ces films peuvent être préparés par la technique du « hand-coating ». Cette technique consiste à déposer sur un support une solution de poly(amide-acide) telle que définie précédemment, le support pouvant être en un matériau tel que du verre. La solution est ensuite séchée, par exemple, à une température de 65 à 80°C, puis soumis à un programme de chauffage, par exemple, à une température de 100 à 400°C, afin d'imidiser le poly(amide-acide) en polyimide. Le support peut être ensuite immergé dans l'eau, permettant au film perdeutéré de se décoller.

Le film peut être caractérisé par spectroscopie infrarouge, la spectroscopie infrarouge permettant notamment de détecter la présence du groupe imide par une bande d'absorption située à 1790 cm⁻¹ et les bandes relatives aux liaisons C-D sortant dans la zone comprise entre 2000 et 2500 cm⁻¹.

Ces films présentent d'excellentes propriétés mécaniques, une excellente résistance à la température ainsi qu'une transparence dans la zone de 2500 à 3500 cm⁻¹.

Enfin, la présente invention a trait à l'utilisation d'un polyimide deutéré conforme à l'invention en tant que matériau transparent dans la zone de 2500 à 3500 cm⁻¹.

L'invention va maintenant être décrite en référence aux exemples suivants donnés à titre illustratif et non limitatif.

### EXPOSÉ DÉTAILLÉ DE MODES DE RÉALISATION PARTICULIERS

Les exemples 1 à 11 illustrent la préparation de polyimides conformes à l'invention.

Chacun de ces exemples illustre la préparation d'un intermédiaire poly(amide-acide) suivie de la transformation de cet intermédiaire en polyimide.

Chacun des polyimides préparés dans ces exemples ont été caractérisés par des essais mécaniques, des essais thermiques et par spectroscopie infra-rouge.

Plus précisément, les polyimides préparés ont été caractérisés mécaniquement par traction sur des éprouvettes normalisées, découpées à l'emporte-pièce de manière à déterminer :
- le module d'Young E, exprimé en Gpa ;
- la contrainte à la rupture, exprimée en Mpa ;
- la déformation à la rupture, exprimée en %.

Les essais thermiques ont consisté à déterminer le coefficient d'expansion thermique (noté CTE et exprimé en 10⁻⁵ K), ce coefficient pouvant être déterminé de deux façons différentes :
- soit par le biais d'un polyimide déposé sous forme de films sur un support (intitulée par la suite «méthode supportée ») ;
- soit par le biais d'un polyimide sous forme de film non supporté (intitulée par la suite « méthode non supportée »).

Enfin, les polyimides préparés ont été caractérisés par spectroscopie IR, de manière à démontrer la totale transparence de ces polyimides dans la zone s'échelonnant de 2500 à 3500 cm⁻¹.

### EXEMPLE 1.

Cet exemple illustre la préparation d'un polyimide comportant un squelette formé d'une alternance entre un motif répétitif de formule (Ia) et un motif répétitif de formule (IIb) :

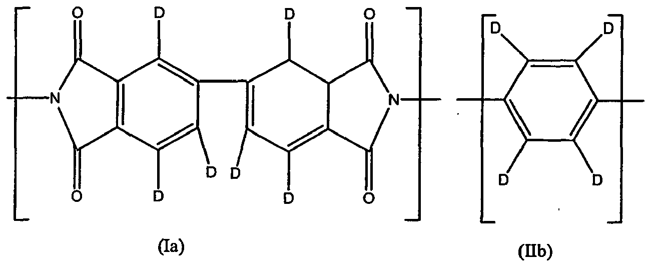

Les réactifs de base sont les suivants :
- le 3,3',4,4'-biphényltétracarboxylique dianhydride-d₆ (abrégé BPDA-d₆) de formule : 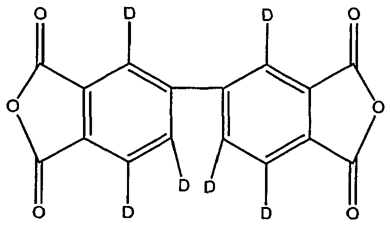
- le p-phénylène diamine-d₈ (abrégé p-PDA-d₈) de formule :

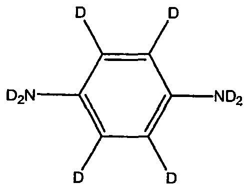

Dans un tricol de 250 mL placé sous un courant d'argon contenant la diamine perdeutérée p-PDA-d₈, purifiée au préalable par sublimation, et mise en solution dans de la N-méthylpyrrolidone (NMP) anhydre, on ajoute progressivement la dianhydride perdeutérée, le BPDA-d₆, en quantités stoechiométriques afin d'atteindre la concentration voulue. Le milieu réactionnel est ensuite laissé sous agitation et à température ambiante durant 20 à 24 heures.

Au terme de cette étape, la solution de poly(amide-acide) obtenue de couleur jaune clair et visqueuse (valeur de viscosité inhérente comprise entre 230 et 280 mL.g⁻¹ sur une solution à 5 g.L⁻¹ et à 30°C) est versée dans un flacon spécifique en verre.

Ensuite, un film de la solution de poly(amide-acide) obtenu est déposé sur une plaque de verre, ladite plaque étant munie de cales d'épaisseur de 20 à 30 µm correspondant à l'épaisseur désirée du film. On place ensuite la plaque de verre sur une plaque thermo-régulée pour réaliser la phase de séchage. Le cycle thermique de séchage est réalisé entre 50 et 80°C par paliers. Le film obtenu après séchage est placé dans une étuve pour réaliser une étape de recuit. Cette étape permet de convertir le film de poly(amide-acide) en polyimide par une réaction de cyclodéshydratation. Le cycle thermique de recuit est compris entre 100 et 300°C avec une vitesse de montée en température de 1 à 5°C par minute. La plaque est ensuite immergée dans un bain d'eau afin de décoller le film en polyimide de la plaque de verre.

Le film obtenu de BPDA-d₆/p-PDA-d₈ subit les analyses suivantes :
- un spectre IR réalisé en transmission ;
- des tests mécaniques conduisant à des valeurs de modules d'Young, de contrainte à la rupture et de déformation à la rupture ;
- une mesure de coefficient d'expansion thermique, selon deux méthodes : la méthode non supportée et la méthode supportée.

Le tableau 1 ci-dessous regroupe les résultats relatifs aux analyses susmentionnées.

**TABLEAU 1.**

| Produit de l'exemple 1 BPDA-d₆ /pPDA-d₈ | Valeur |
|---|---|
| Module de Young E (en Gpa) | 8 |
| Contrainte à la rupture σᵣ (en Mpa) | 335 |
| Déformation à la rupture εᵣ (en %) | 20 |
| Coefficient d'expansion thermique (10⁻⁵ °K) | 0,4-1,7 |
| Longueur d'onde de vibration des liaisons C-D (en cm⁻¹) | 2247 |

Ainsi, le spectre IR du produit de l'exemple 1 présente un pic d'absorption à 2247 cm⁻¹ correspondant à l'absorption d'une liaison carbone-deutérium aromatique et ne présente pas de pics d'absorption correspondant aux liaisons C-H aromatiques à 3080 cm⁻¹. Ce produit présente une totale transparence (c'est-à-dire aucune atténuation optique) dans la zone de 2500 à 3500 cm⁻¹.

Ce produit présente également d'excellentes propriétés mécaniques (contrainte à la rupture de 335 MPa) par rapport à des produits de l'art antérieur.

### EXEMPLE 2.

Cet exemple illustre la préparation d'un polyimide dont le squelette est formé d'une alternance entre un motif répétitif de formule (Ia) et un motif répétitif de formule (IIc) :

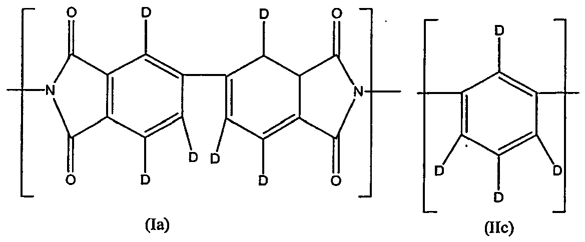

Les réactifs de base sont les suivants :
- le 3,3',4,4'-biphényltétracarboxylique dianhydride-d₆ (abrégé BPDA-d₆) de formule : 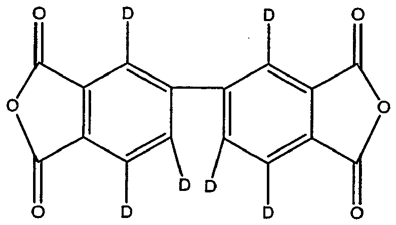
- le m-phénylène diamine-d₈ (abrégé m-PDA-d₈) de formule :

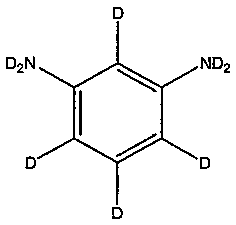

Le polyimide décrit ci-dessus, intitulé BPDA-d₆/m-PDA-d₈, est préparé selon le même mode opératoire que celui de l'exemple 1 et subit les mêmes analyses.

Le tableau 2 ci-dessous regroupe les résultats relatifs aux analyses susmentionnées.

**TABLEAU 2**

| Produit de l'exemple 2 BPDA-d_{6/}m-PDA-d₈ | Valeur |
|---|---|
| Module de Young E (en Gpa) | 8 |
| Contrainte à la rupture σᵣ (en Mpa) | 340 |
| Déformation à la rupture εᵣ (en %) | 20 |
| Coefficient d'expansion thermique (10⁻⁵ °K) | 0,1-1,9 |
| Longueur d'onde de vibration des liaisons C-D (en cm⁻¹) | 2255 |

Ce produit présente une transparence dans la zone de 2500 à 3500 cm⁻¹.

Ce produit présente également d'excellentes propriétés mécaniques (telles qu'une contrainte à la rupture de 340 MPa) par rapport à des produits de l'art antérieur.

### EXEMPLE 3.

Cet exemple illustre la préparation d'un polyimide dont le squelette est formé d'une alternance entre un motif répétitif de formule (Ia) et un motif répétitif de formule (IId) :

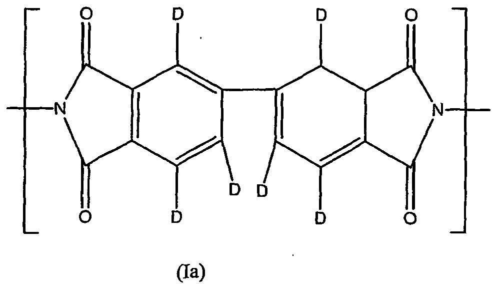

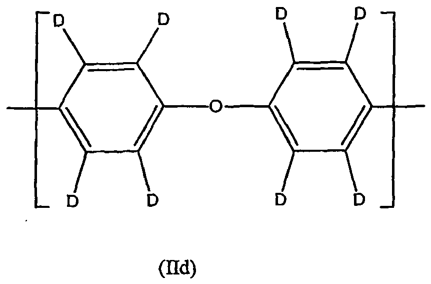

Les réactifs de base sont les suivants :
- le 3,3',4,4'-biphényltétracarboxylique dianhydride-d₆ (abrégé BPDA-d₆) de formule : 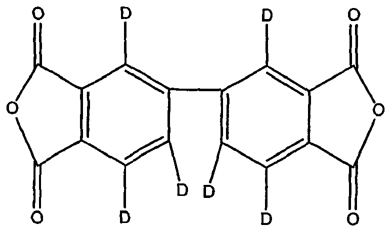
- l'oxydianiline-d₁₂ (abrégé ODA-d₁₂) de formule :

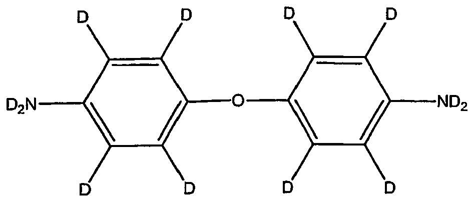

Le polyimide décrit ci-dessus, intitulé BPDA-d₆/ODA-d₁₂, est préparé selon le même mode opératoire que celui de l'exemple 1 et subit les mêmes analyses.

Le tableau 3 ci-dessous regroupe les résultats relatifs aux analyses susmentionnées.

**TABLEAU 3**

| Produit de l'exemple 3 BPDA-d₆/ODA-d₁₂ | Valeur |
|---|---|
| Module de Young E (en Gpa) | 4 |
| Contrainte à la rupture σᵣ (en Mpa) | 120 |
| Déformation à la rupture εᵣ (en %) | 20 |
| Coefficient d'expansion thermique (10⁻⁵ °K) | 1,7-5,0 |
| Longueur d'onde de vibration des liaisons C-D (en cm⁻¹) | 2254 |

Ce produit présente une transparence dans la zone de 2500 à 3500 cm⁻¹.

Ce produit présente également de meilleures propriétés mécaniques (contrainte à la rupture de 120 MPa) que les produits de l'art antérieur.

### EXEMPLE 4.

Cet exemple illustre la préparation d'un polyimide dont le squelette est formé d'une alternance entre un motif répétitif de formule (Ib) et un motif répétitif de formule (IIb) :

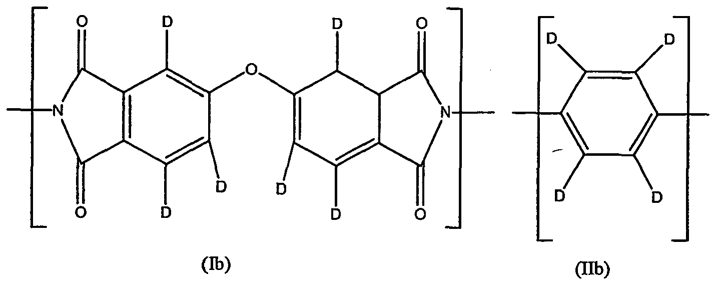

Les réactifs de base sont les suivants :
- le dianhydride bis(3,4-dicarboxyphénylène)éther-d₆ (abrégé ODPA-d₆) de formule: 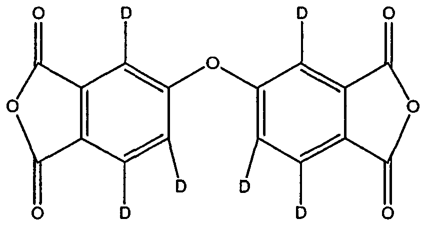
- le p-phénylène diamine-d₈ (abrégé p-PDA-d₈) de formule :

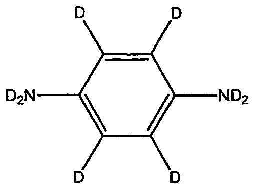

Le polyimide décrit ci-dessus, intitulé ODPA-d₆/PDA-d₈, est préparé selon le même mode opératoire que celui de l'exemple 1 et subit les mêmes analyses.

Le tableau 4 ci-dessous regroupe les résultats relatifs aux analyses susmentionnées.

**TABLEAU 4**

| Produit de l'exemple 4 ODPA-d_{6/}PDA-d₈ | Valeur |
|---|---|
| Module de Young E (en Gpa) | 6 |
| Contrainte à la rupture σᵣ (en Mpa) | 180 |
| Déformation à la rupture εᵣ (en %) | 15 |
| Coefficient d'expansion thermique (10⁻⁵ °K) | 2,6 |
| Longueur d'onde de vibration des liaisons C-D (en cm⁻¹) | 2260 |

Ce produit présente une transparence dans la zone de 2500 à 3500 cm⁻¹.

Ce produit présente également de très bonnes propriétés mécaniques (contrainte à la rupture de 180 MPa) par rapport à des produits de l'art antérieur.

### EXEMPLE 5.

Cet exemple illustre la préparation d'un polyimide dont le squelette est formé d'une alternance entre un motif répétitif de formule (Ib) et un motif répétitif de formule (IId) :

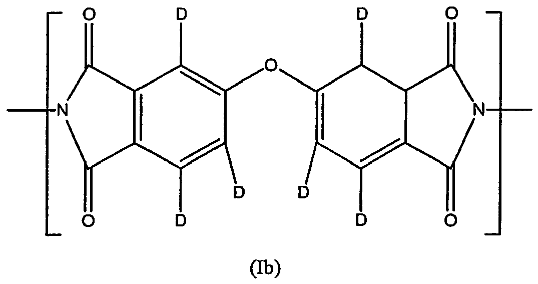

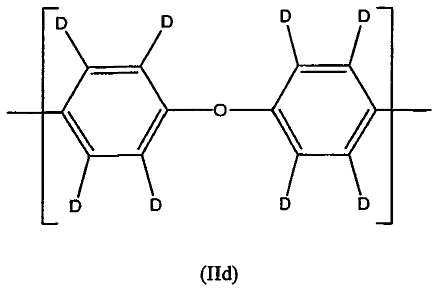

Les réactifs de base sont les suivants :
- le dianhydride bis(3,4-dicarboxyphénylène)éther-d₆ (abrégé ODPA-d₆) de formule: 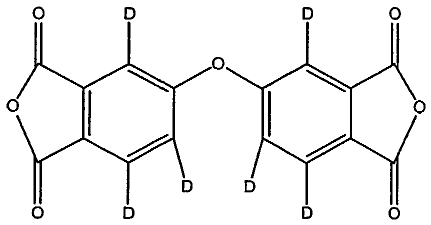
- l'oxydianiline-d₁₂ (abrégé ODA-d₁₂) de formule :

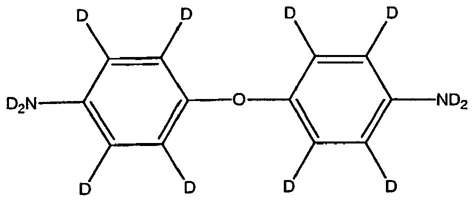

Le polyimide décrit ci-dessus, intitulé ODPA-d₆/ODA-d₈, est préparé selon le même mode opératoire que celui de l'exemple 1 et subit les mêmes analyses.

Le tableau 5 ci-dessous regroupe les résultats relatifs aux analyses susmentionnées.

**TABLEAU 5**

| Produit de l'exemple 5 ODPA-d_{6/}ODA-d₈ | Valeur |
|---|---|
| Module de Young E (en Gpa) | 3 |
| Contrainte à la rupture σᵣ (en Mpa) | 140 |
| Déformation à la rupture εᵣ (en %) | 70 |
| Coefficient d'expansion thermique (10⁻⁵ °K) | 4 |
| Longueur d'onde de vibration des liaisons C-D (en cm⁻¹) | 2255 |

Ce produit présente une transparence dans la zone de 2500 à 3500 cm⁻¹.

Ce produit présente également de meilleures propriétés mécaniques (contrainte à la rupture de 140 MPa) que les produits de l'art antérieur.

### EXEMPLE 6.

Cet exemple illustre la préparation d'un polyimide, dont le squelette est formé d'une alternance entre un motif répétitif de formule (Ic) et un motif répétitif de formule (IIb) :

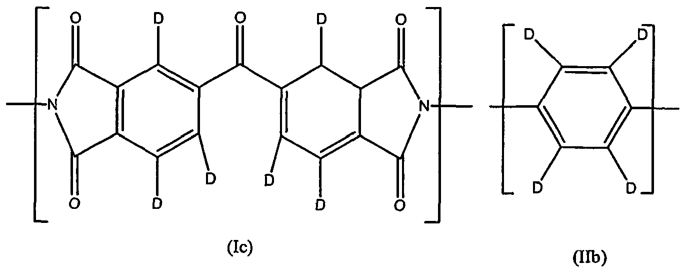

Les réactifs de base sont les suivants :
- le dianhydride-3,3',4,4'-benzophénone-d₆ (abrégé BTDA-d₆) de formule : 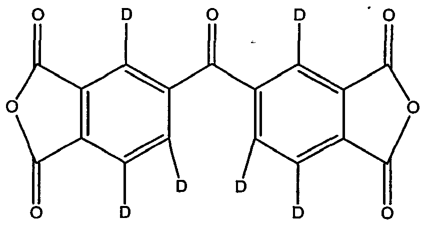
- le p-phénylène diamine-d₈ (abrégé p-PDA-d₈) de formule :

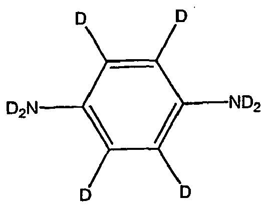

Le polyimide décrit ci-dessus, intitulé BTDA-d₆/pPDA-d₈, est préparé selon le même mode opératoire que celui de l'exemple 1 et subit les mêmes analyses.

Le tableau 6 ci-dessous regroupe les résultats relatifs aux analyses susmentionnées.

**TABLEAU 6**

| Produit de l'exemple 6 BTDA-d₆/pPDA-d₈ | Valeur |
|---|---|
| Module de Young E (en Gpa) | 7 |
| Contrainte à la rupture σᵣ (en Mpa) | 175 |
| Déformation à la rupture εᵣ (en %) | 10 |
| Coefficient d'expansion thermique (10⁻⁵ °K) | 1,7-4 |
| Longueur d'onde de vibration des liaisons C-D (en cm⁻¹) | 2251 |

Ce produit présente une transparence dans la zone de 2500 à 3500 cm⁻¹.

Ce produit présente également de très bonnes propriétés mécaniques (contrainte à la rupture de 175 MPa) par rapport à des produits de l'art antérieur.

### EXEMPLE 7.

Cet exemple illustre la préparation d'un polyimide dont le squelette est formé d'une alternance entre un motif répétitif de formule (Ic) et un motif répétitif de formule (IId) :

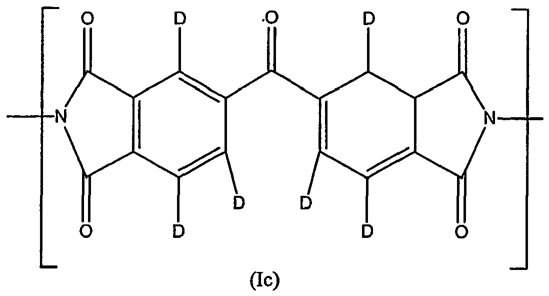

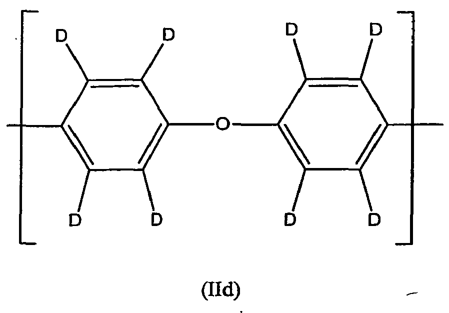

Les réactifs de base sont les suivants :
- le dianhydride-3,3',4,4'-benzophénone-d₆ (abrégé BTDA-d₆) de formule : 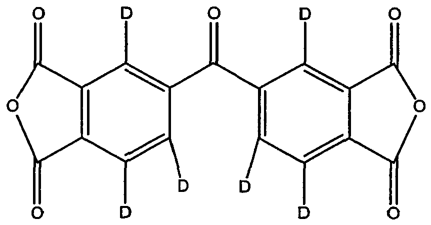
- l'oxydianiline-d₁₂ (abrégé ODA-d₁₂) de formule :

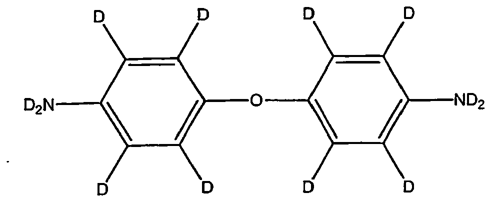

Le polyimide décrit ci-dessus, intitulé BTDA-d₆/ODA-d₁₂, est préparé selon le même mode opératoire que celui de l'exemple 1 et subit les mêmes analyses.

Le tableau 7 ci-dessous regroupe les résultats relatifs aux analyses susmentionnées.

**TABLEAU 7**

| Produit de l'exemple 7 BTDA-d₆/ODA-d₁₂ | Valeur |
|---|---|
| Module de Young E (en Gpa) | 3 |
| Contrainte à la rupture σᵣ (en Mpa) | 135 |
| Déformation à la rupture εᵣ (en %) | 60 |
| Coefficient d'expansion thermique (10⁻⁵ °K) | 3,5-5 |
| Longueur d'onde de vibration des liaisons C-D (en cm⁻¹) | 2256 |

Ce produit présente une transparence dans la zone de 2500 à 3500 cm⁻¹.

Ce produit présente également de meilleures propriétés mécaniques que des produits de l'art antérieur.

### EXEMPLE 8.

Cet exemple illustre la préparation d'un polyimide dont le squelette est formé d'une alternance entre un motif répétitif de formule (Id) et un motif répétitif de formule (IId) :

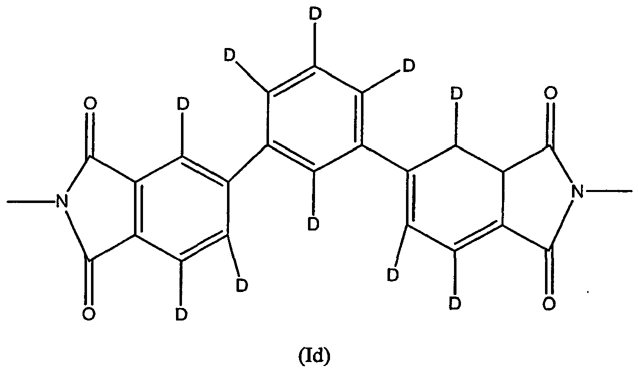

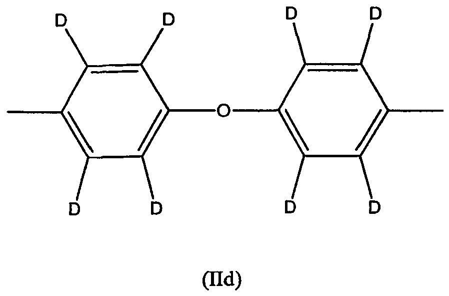

Les réactifs de base sont les suivants :
- le dianhydride-3,3",4,4"-m-terphénylique-d₁₀ (abrégé MTPDA-d₁₀) de formule : 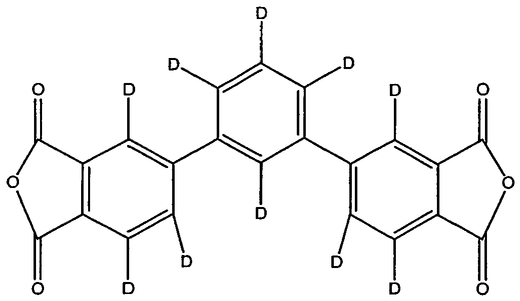
- l'oxydianiline-d12 (abrégé ODA-d₁₂) de formule :

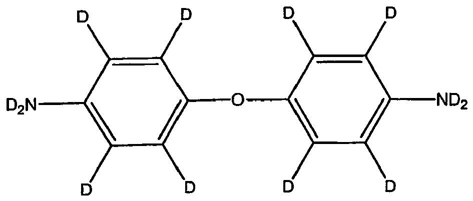

Le polyimide décrit ci-dessus, intitulé MTPDA-d₁₀/ODA-d₈, est préparé selon le même mode opératoire que celui de l'exemple 1 et subit les mêmes analyses.

Le tableau 8 ci-dessous regroupe les résultats relatifs aux analyses susmentionnées.

**TABLEAU 8**

| Produit de l'exemple 8 MTPDA-d₁₀/ODA-d₈ | Valeur |
|---|---|
| Module de Young E (en Gpa) | 3 |
| Contrainte à la rupture σᵣ (en Mpa) | 130 |
| Déformation à la rupture εᵣ (en %) | 40 |
| Coefficient d'expansion thermique (10⁻⁵ °K) | 2-4 |
| Longueur d'onde de vibration des liaisons C-D (en cm⁻¹) | 2240 |

Ce produit présente une transparence dans la zone de 2500 à 3500 cm⁻¹.

Ce produit présente également de meilleures propriétés mécaniques (contrainte à la rupture de 130 Mpa) que des produits de l'art antérieur.

### EXEMPLE 9.

Cet exemple illustre la préparation d'un polyimide comprenant un motif répétitif de formule (Ia), un motif répétitif de formule (IIb) et un motif répétitif de formule (III) :

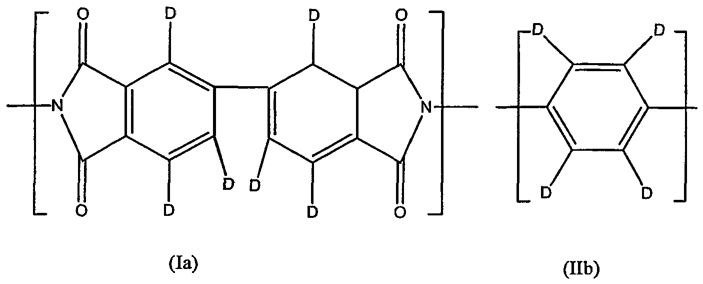

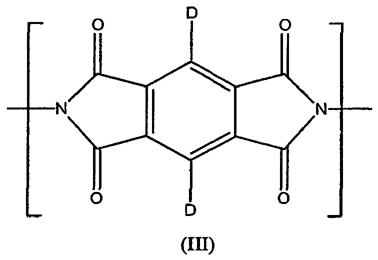

Les réactifs de base sont les suivants :
- le 3,3',4,4'-biphényltétracarboxylique dianhydride-d₆ (abrégé BPDA-d₆) de formule : 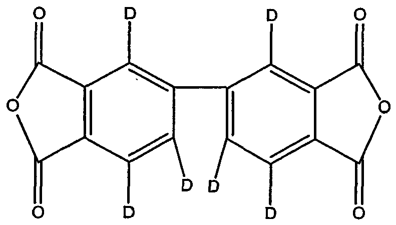
- le p-phénylène diamine-d₈ (abrégé p-PDA-d₈) de formule : 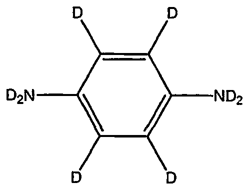
- le dianhydride pyromélittique deutéré-d₂ (PMDa-d₂) de formule:

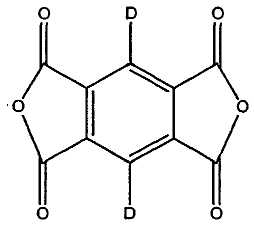

Le polyimide décrit ci-dessus, intitulé BPDA-d₆-PMDA-d₂-pPDA-d₈, est préparé selon le même mode opératoire que celui des l'exemple 1 et subit les mêmes analyses.

Le tableau 9 ci-dessous regroupe les résultats relatifs aux analyses susmentionnées.

**TABLEAU 9**

| Produit de l'exemple 9 BPDA-d₆-PMDA-d₂-pPDA-d₈ | Valeur |
|---|---|
| Module de Young E (en Gpa) | 8 |
| Contrainte à la rupture σᵣ (en Mpa) | 300 |
| Déformation à la rupture εᵣ (en %) | 25 |
| Coefficient d'expansion thermique (10⁻⁵ °K) | 1,3 |
| Longueur d'onde de vibration des liaisons C-D (en cm⁻¹) | 2257 |

Ce produit présente une transparence dans la zone de 2500 à 3500 cm⁻¹.

Ce produit présente également d'excellentes propriétés mécaniques (contrainte à la rupture 300 MPa) par rapport à des produits de l'art antérieur.

### EXEMPLE 10.

Cet exemple illustre la préparation d'un polyimide comprenant un motif répétitif de formule (Ia), un motif répétitif de formule (IIb) et un motif répétitif de formule (IId) :

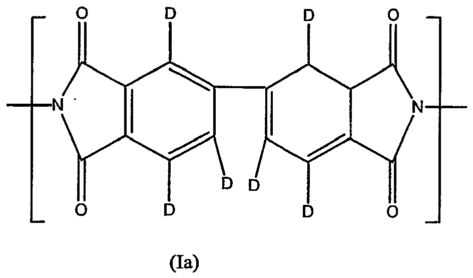

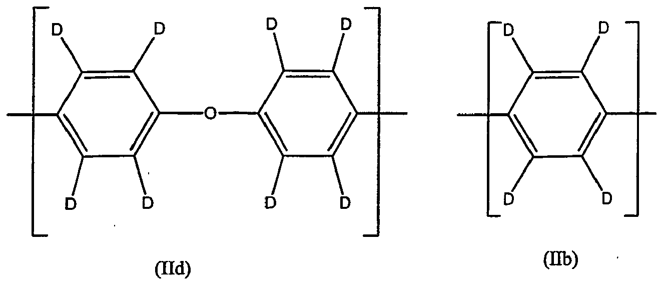

Les réactifs de base sont les suivants :
- le 3,3',4,4'-biphényltétracarboxylique dianhydride-d₆ (abrégé BPDA-d₆) de formule : 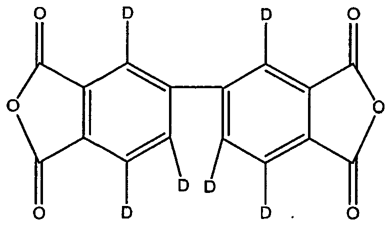
- le p-phénylène diamine-d₈ (abrégé p-PDA-d₈) de formule : 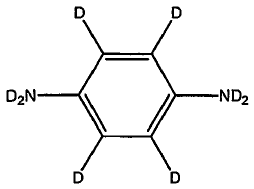
- l'oxydianiline-d₁₂ (abrégé ODA-d₁₂) de formule :

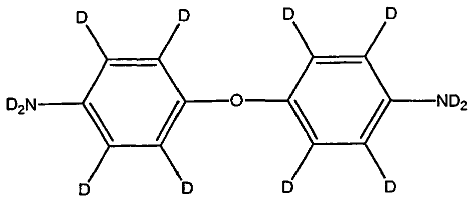

Le polyimide décrit ci-dessus, intitulé BPDA-d₆-pPDA-d₈-oDA-d₁₂, est préparé selon le même mode opératoire que celui de l'exemple 1 et subit les mêmes analyses.

Le tableau 10 ci-dessous regroupe les résultats relatifs aux analyses susmentionnées.

**TABLEAU 10**

| Produit de l'exemple 10 BPDA-d₆-pPDA-d₈-ODA-d₁₂ | Valeur |
|---|---|
| Module de Young E (en Gpa) | 7 |
| Contrainte à la rupture σᵣ (en Mpa) | 210 |
| Déformation à la rupture εᵣ (en %) | 35 |
| Coefficient d'expansion thermique (10⁻⁵ °K) | 0,5 |
| Longueur d'onde de vibration des liaisons C-D (en cm⁻¹) | 2255 |

Ce produit présente une transparence dans la zone de 2500 à 3500 cm⁻¹.

Ce produit présente également de très bonnes propriétés mécaniques (contrainte à la rupture de 210 MPa) par rapport à des produits de l'art antérieur.

### EXEMPLE 11.

Cet exemple illustre la préparation d'un polyimide comprenant un motif répétitif de formule (Ic), un motif répétitif de formule (IIb) et un motif répétitif de formule (IId) :

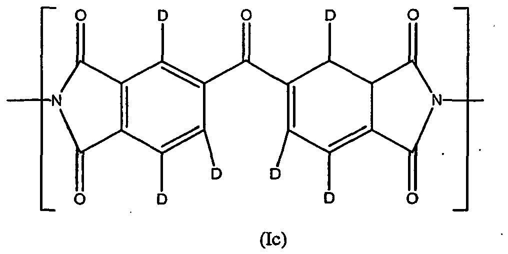

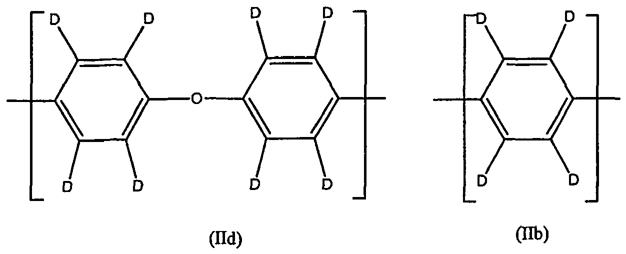

Les réactifs de base sont les suivants :
- le dianhydride-3,3',4,4'-benzophénone-d₆ (abrégé BTDA-d₆) de formule : 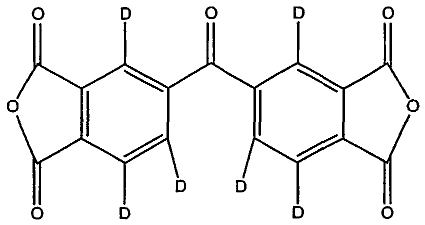
- le p-phénylène diamine-d₈ (abrégé p-PDA-d₈) de formule : 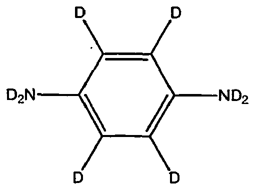
- l'oxydianiline-d₁₂ (abrégé ODA-d₁₂) de formule :

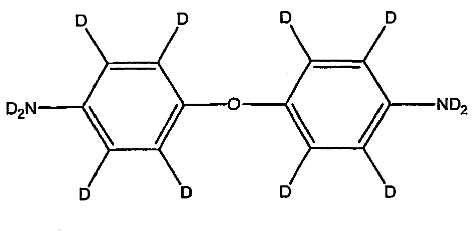

Le polyimide décrit ci-dessus, intitulé BTDA-d₂-_{P}PDA-d₈-ODA-d₁₂, est préparé selon le même mode opératoire que celui de l'exemple 1 et subit les mêmes analyses.

Le tableau 11 ci-dessous regroupe les résultats relatifs aux analyses susmentionnées.

**TABLEAU 11**

| Produit de l'exemple 11 BTDA-d₂-pPDA-d₈-ODA-d₁₂ | Valeur |
|---|---|
| Module de Young E (en Gpa) | 5 |
| Contrainte à la rupture σᵣ (en Mpa) | 145 |
| Déformation à la rupture εᵣ (en %) | 25 |
| Coefficient d'expansion thermique (10⁻⁵ °K) | 2 |
| Longueur d'onde de vibration des liaisons C-D (en cm⁻¹) | 2262 |

Ce produit présente une transparence dans la zone de 2500 à 3500 cm⁻¹.

Ce produit présente également de meilleures propriétés mécaniques que des produits de l'art antérieur.

### Références citées.

[1] Kitano, Reports on Progress in Polymer Physics in Japan, vol 43, 2000 ;
[2] Saint-Clair et al., Sampe Journal, August 1985, pp 28-33 ;
[3] US 5,233,018 ;
[4] US 6,048,986 ;
[5] Wallace et al., Nuclear Instruments and Methods in Physics Research B 103 (1995),435-439 ;
[6] Werstiuk et al., Can.J.Chem, vol 52, 2169-2171, 1973 ;
[7] Artamkina et al., traduit de Zhurnal Organicheskoi Khimii, vol.16, n°4, 99.698-702, April 1980.

## Revendications

1. Polyimide deutéré, dont le squelette comprend une alternance entre:
- au moins un motif répétitif répondant à la formule (I) suivante : 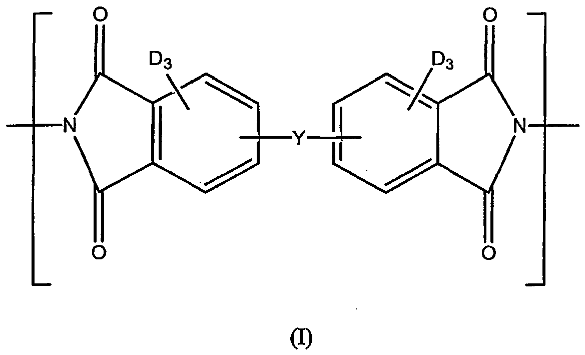 dans laquelle :
- Y représente une liaison simple ou un groupe espaceur ; et
- au moins un motif répétitif répondant à la formule (II) suivante : 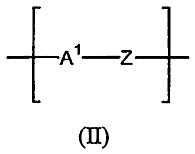 dans laquelle :
- A¹ représente un groupe aromatique perdeutéré comportant de 6 à 10 atomes de carbone ; et
- Z représente une liaison simple ou un groupe choisi parmi -O-C₆D₄-, -CO-C₆D₄-, -C₆D₄-.

2. Polyimide deutéré selon la revendication 1, dans lequel Y, lorsque Y est un groupe espaceur, est un groupe choisi parmi -O-, -CD₂-, -CO-, -SO₂-, -C₆D₄-.

3. Polyimide deutéré selon la revendication 1 ou 2, dans lequel le motif répétitif conforme à la formule (II) est un motif répétitif de formule (IIa) suivante: 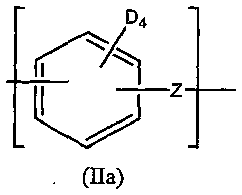 dans laquelle Z répond à la même définition que celle donnée dans la revendication 1.

4. Polyimide deutéré selon l'une quelconque des revendications 1 à 3, choisi dans le groupe constitué des polyimides choisis parmi :
- les polyimides comprenant un motif de formule (Ia) et un motif répétitif de formule (IIb) suivantes : 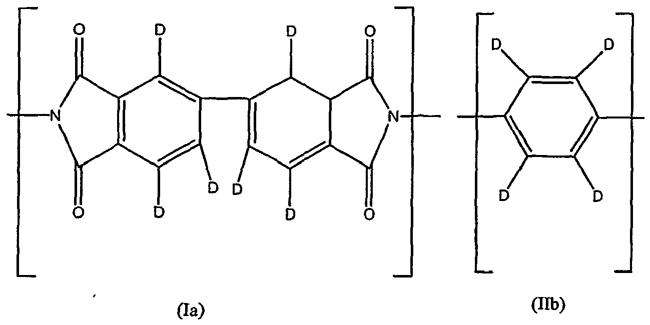
- les polyimides comprenant un motif répétitif de formule (Ia) et un motif répétitif de formule (IIc) suivantes : 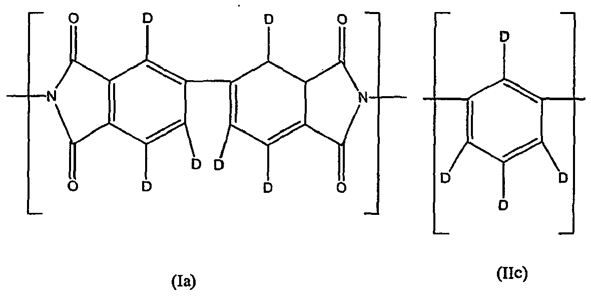
- les polyimides comprenant un motif répétitif de formule (Ia) et un motif répétitif de formule (IId) suivantes : 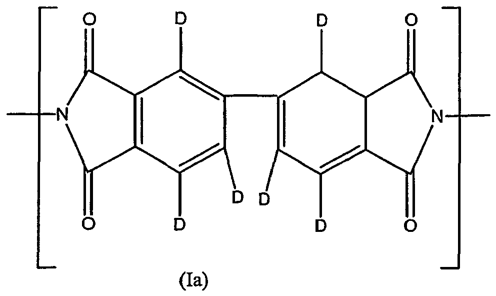 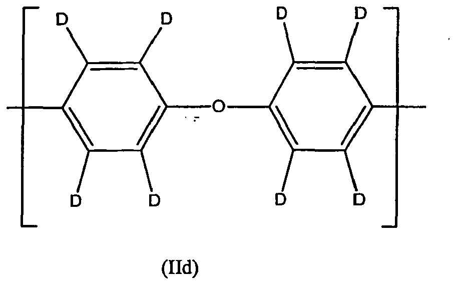
- les polyimides comprenant un motif répétitif de formule (Ib) et un motif répétitif de formule (IIb) suivantes : 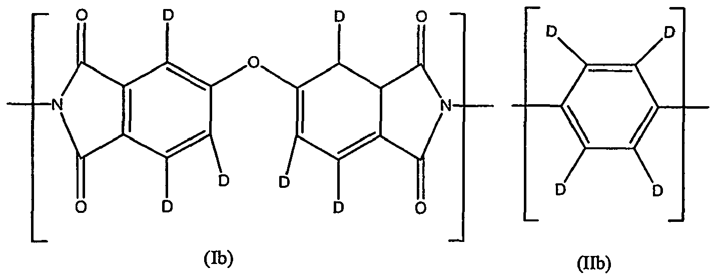
- les polyimides comprenant un motif répétitif de formule (Ib) et un motif répétitif de formule (IId) suivantes : 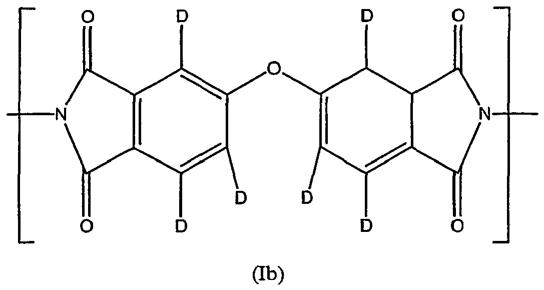 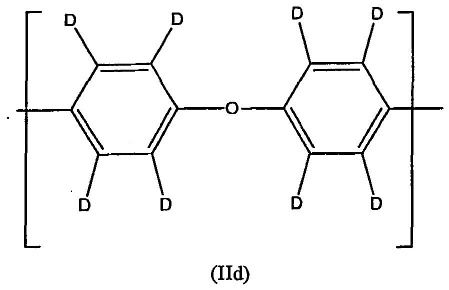
- les polyimides comprenant un motif répétitif de formule (Ic) et un motif répétitif de formule (IIb) suivantes : 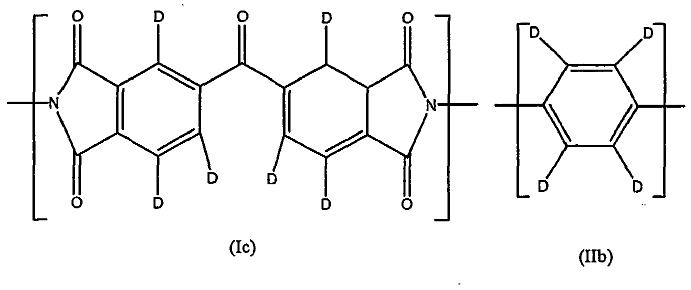
- les polyimides comprenant un motif répétitif de formule (Ic) et un motif répétitif de formule (IId) suivantes : 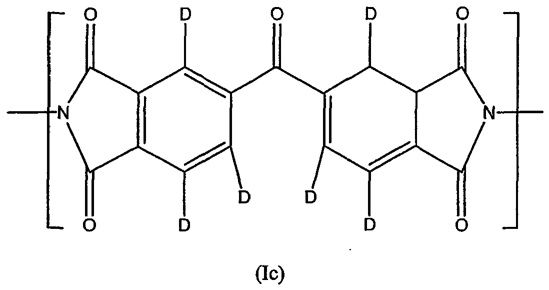 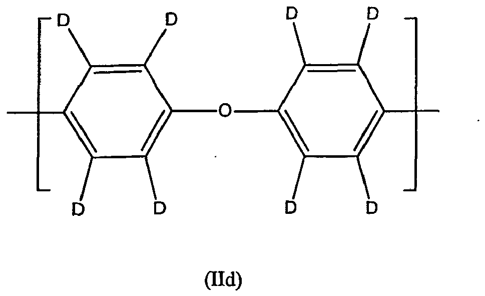
- les polyimides comprenant un motif répétitif de formule (Id) et un motif répétitif de formule (IId) suivantes : 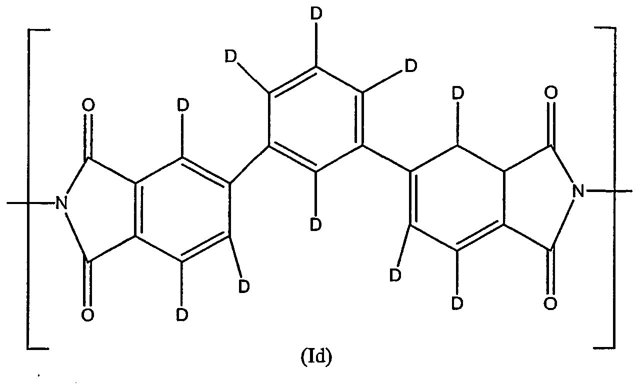 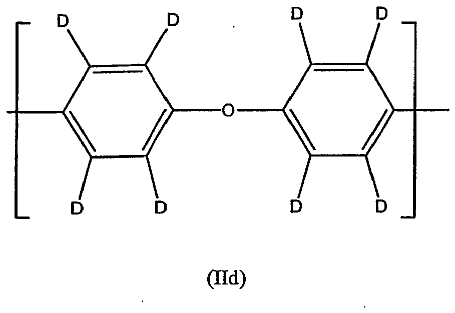
- les polyimides comprenant un motif répétitif de formule (Ia), un motif répétitif de formule (IIb) et un motif répétitif de formule (IId) suivantes : 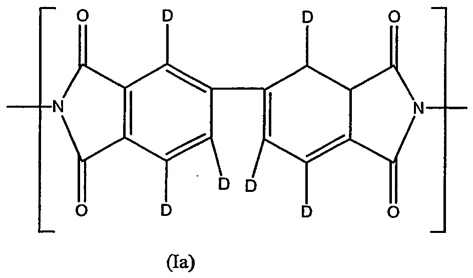 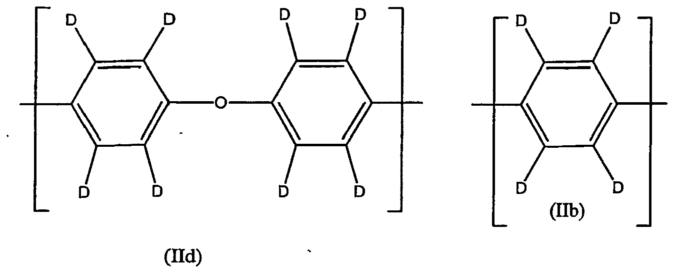
- les polyimides comprenant un motif répétitif de formule (Ic), un motif répétitif de formule (IIb) et un motif répétitif de formule (IId) suivantes :

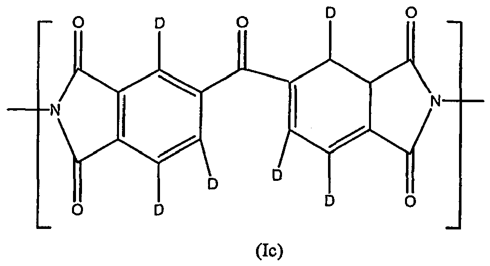

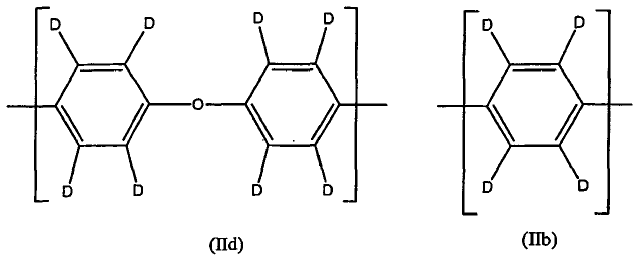

5. Polyimide deutéré selon l'une quelconque des revendications 1 à 3, comprenant en outre un motif répondant à la formule (III) suivante :

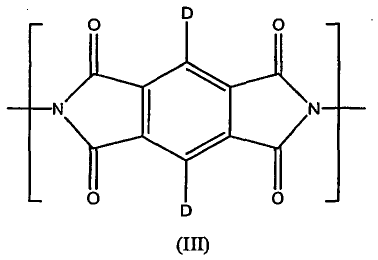

6. Polyimide deutéré selon la revendication 5, comprenant un motif répétitif de formule (Ia), un motif répétitif de formule (IIb) et un motif répétitif de formule (III) suivantes :

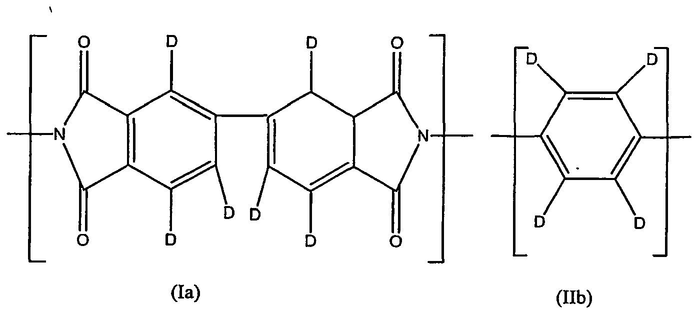

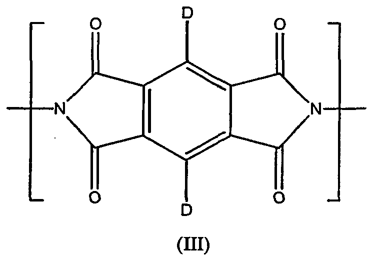

7. Procédé de préparation d'un polyimide deutéré tel que défini selon l'une quelconque des revendications 1 à 3, ledit procédé comprenant une étape consistant à traiter par un chauffage à une température adéquate une solution d'un poly(amide-acide), dont le squelette comprend une alternance entre au moins un motif répétitif de formule (IV) suivante : 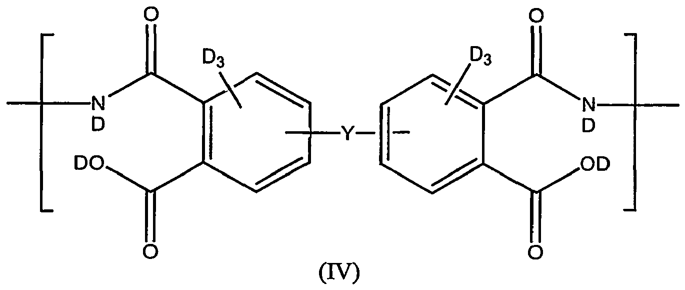 dans laquelle Y répond à la même définition que celle donnée dans la revendication 1 ; et
au moins un motif répétitif de formule (II) : 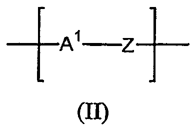 dans laquelle A¹ et Z répondent aux mêmes définitions que celles données dans la revendication 1,
la température de chauffage adéquate étant déterminée de manière à obtenir une imidisation totale dudit poly(amide-acide).

8. Procédé de préparation selon la revendication 7, dans lequel la température de chauffage adéquate est une température allant de 80 à 400 °C.

9. Procédé de préparation selon la revendication 7 ou 8, dans lequel la solution de poly(amide-acide) est préparée par polycondensation dans un solvant d'au moins un monomère de formule (V) suivante : 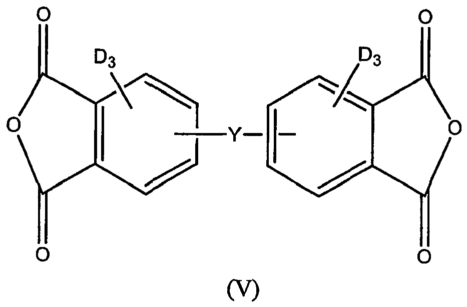 dans laquelle Y répond à la même définition que celle donnée dans la revendication 1, et
d'au moins un monomère de formule (VI) suivante:
ND₂-A¹-Z-ND₂ (VI)
dans laquelle A¹ et Z répondent aux mêmes définitions que celles données dans la revendication 1.

10. Procédé de préparation selon l'une quelconque des revendications 7 à 9, dans lequel le solvant est un solvant dipolaire aprotique choisi dans le groupe constitué par la N-méthylpyrrolidone (NMP), le diméthylformamide (DMF) et le diméthylacétamide (DMAC).

11. Monomère dianhydride deutéré répondant à la formule (V) suivante : 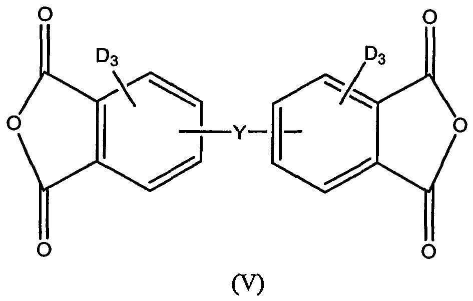 dans laquelle Y répond à la même définition que celle donnée dans la revendication 1.

12. Monomères dianhydrides deutérés répondant à l'une des formules suivantes :

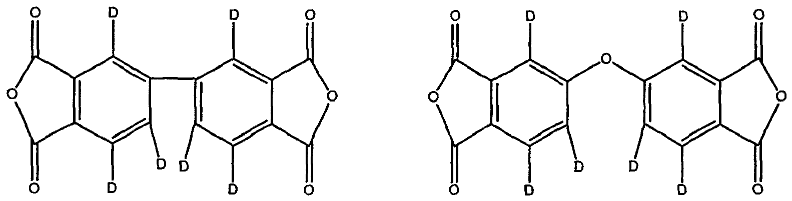

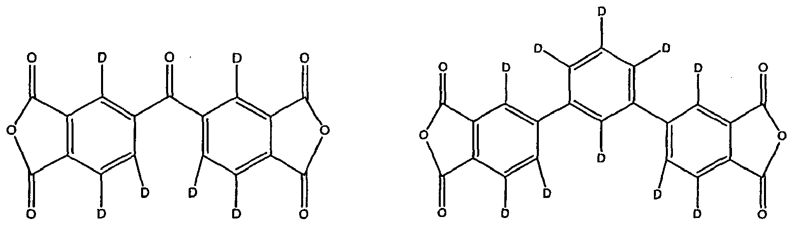

13. Procédé de préparation de monomères de formule (V): 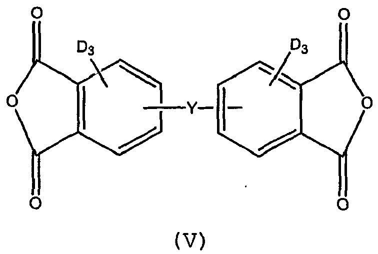 dans laquelle Y répond à la même définition que celle donnée dans la revendication 1, ledit procédé comprenant successivement les étapes suivantes:
- soumettre un composé de formule (VII) : 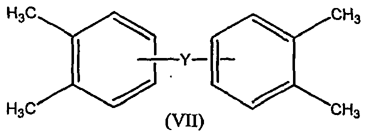 à une deutération de façon à obtenir un composé de formule (VIII): 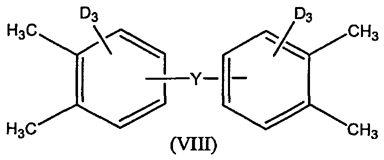
- soumettre le composé précédemment obtenu à une oxydation, de manière à obtenir un composé de formule (IX) : 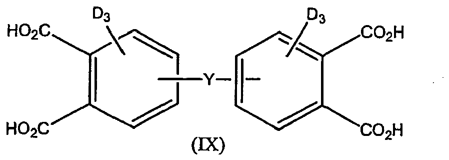
- soumettre le composé précédemment obtenu à une cyclodéshydratation, de façon à obtenir le composé de formule (V).

14. Procédé de préparation de monomères diamines deutérés de formule (VI) : 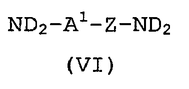 dans laquelle A¹ et Z répondent aux mêmes définitions que celles données dans la revendication 1, ledit procédé comprenant successivement les étapes suivantes:
- faire réagir un composé de formule (XI): 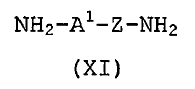 avec un acide minéral de formule HX de façon à obtenir un sel d'ammonium de formule (XII) : 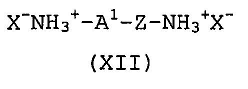 dans laquelle X représente un halogénure;
- faire réagir ledit sel d'ammonium avec de l'eau deutérée sous une pression adéquate, suivie d'une réaction avec une base de manière à obtenir le monomère de formule (VI).

15. Film à base d'un polyimide deutéré tel que défini selon l'une quelconque des revendications 1 à 6.

16. Utilisation d'un polyimide deutéré tel que défini selon l'une quelconque des revendications 1 à 6 en tant que matériau transparent dans la zone de 2500 à 3500 cm⁻¹.

## Claims

1. A deuterated polyimide, the backbone of which comprises an alternation between:
- at least one repeat unit corresponding to the following formula (I): 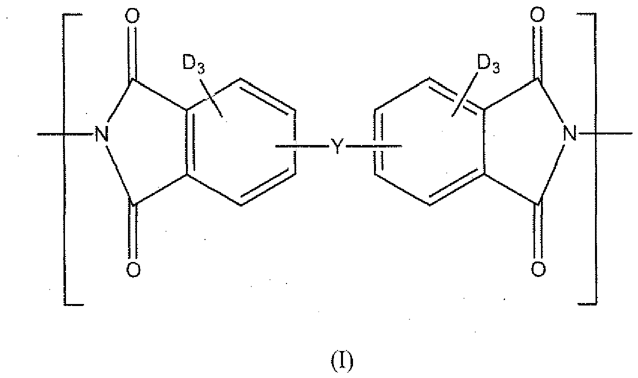 in which:
- Y represents a single bond or a spacer group; and
- at least one repeat unit corresponding to the following formula (II): 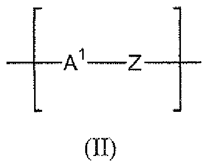 in which:
- A¹ represents a perdeuterated aromatic group comprising from 6 to 10 carbon atoms; and
- Z represents a single bond or a group chosen from -O-C₆D₄-, -CO-C₆D₄- and -C₆D₄-.

2. The deuterated polyimide as claimed in claim 1, in which Y, when Y is a spacer group, is a group chosen from -O-, -CD₂-, -CO-, -SO₂- or -C₆D₄-.

3. The deuterated polyimide as claimed in claim 1 or 2, in which the repeat unit in accordance with the formula (II) is a repeat unit of following formula (IIa): 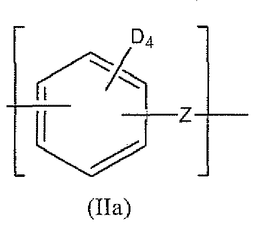 in which Z corresponds to the same definition as that given in claim 1.

4. The deuterated polyimide as claimed in any one of claims 1 to 3, chosen from the group consisting of the polyimides chosen from:
- polyimides comprising a repeat unit of following formula (Ia) and a repeat unit of following formula (IIb): 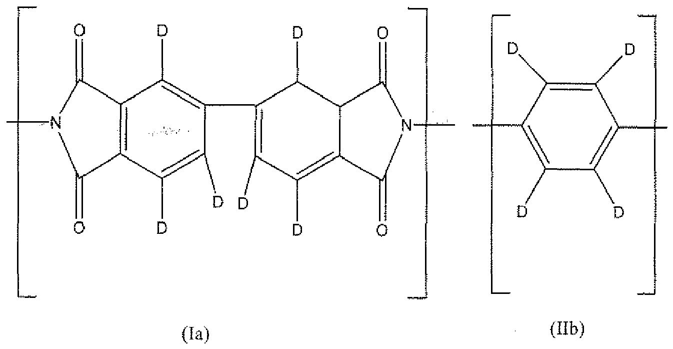
- polyimides comprising a repeat unit of following formula (Ia) and a repeat unit of following formula (IIc) : 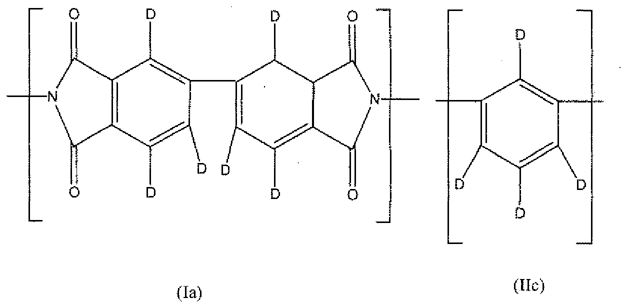
- polyimides comprising a repeat unit of following formula (Ia) and a repeat unit of following formula (IId) : 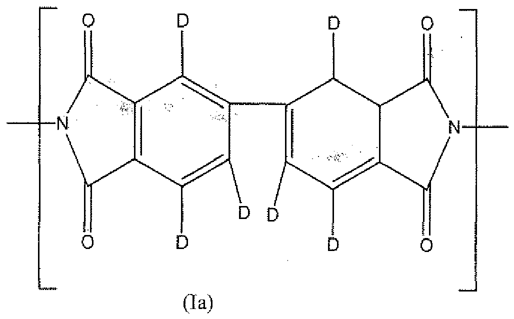 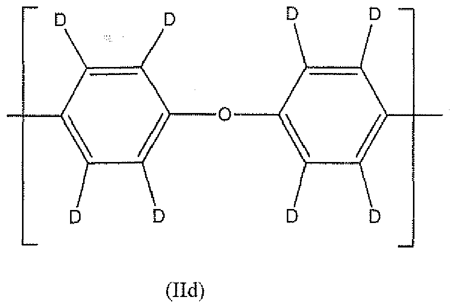
- polyimides comprising a repeat unit of following formula (Ib) and a repeat unit of following formula (IIb) : 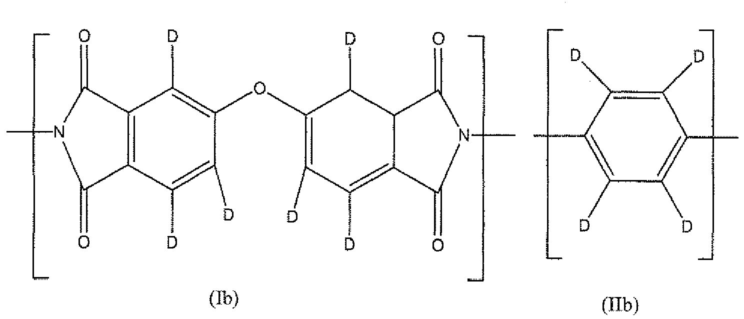
- polyimides comprising a repeat unit of following formula (Ib) and a repeat unit of following formula (IId): 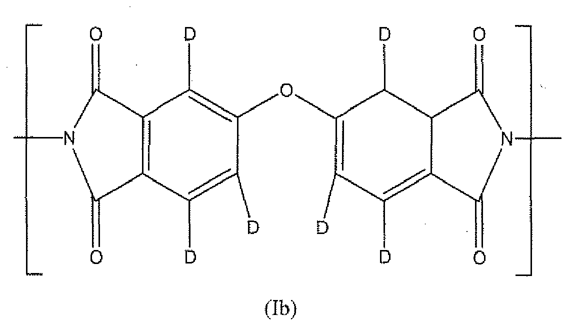 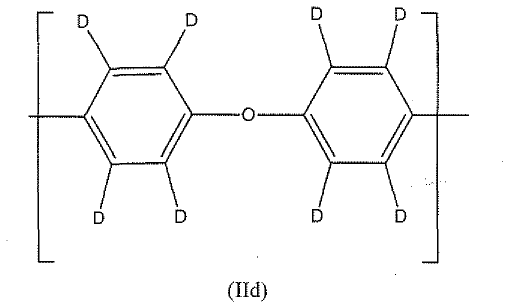
- polyimides comprising a repeat unit of following formula (Ic) and a repeat unit of following formula (IIb) : 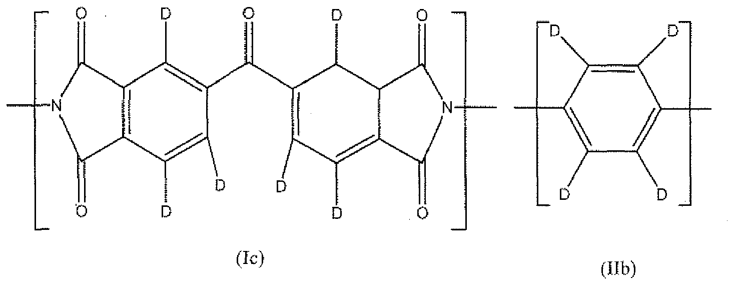
- polyimides comprising a repeat unit of following formula (Ic) and a repeat unit of following formula (IId) : 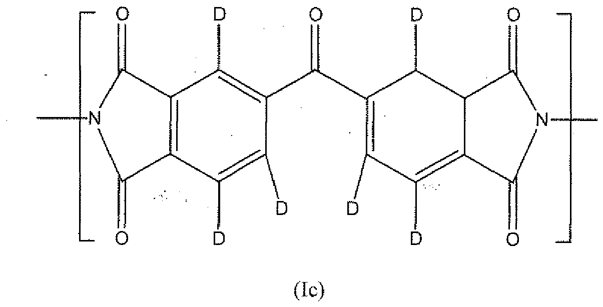 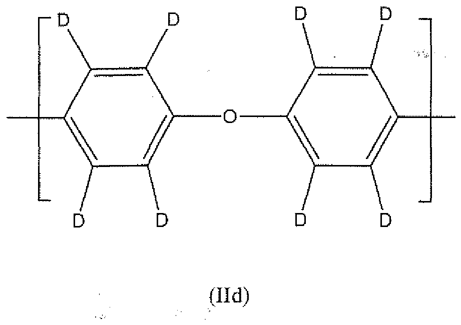
- polyimides comprising a repeat unit of following formula (Id) and a repeat unit of following formula (IId) : 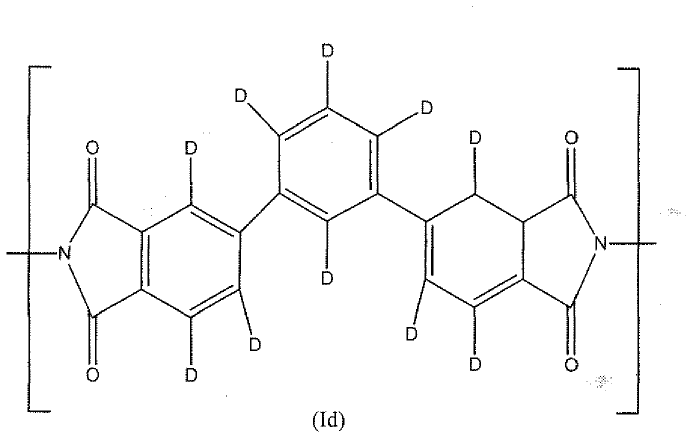 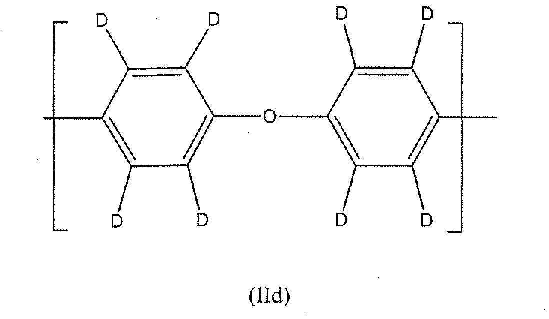
- polyimides comprising a repeat unit of following formula (Ia), a repeat unit of following formula (IIb) and a repeat unit of following formula (IId) : 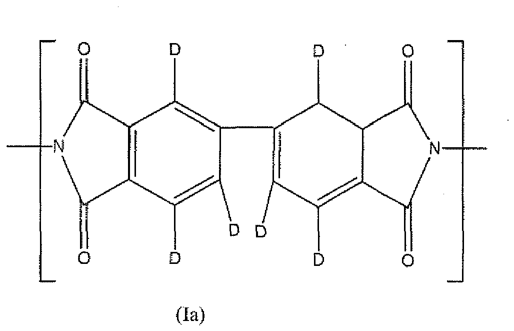 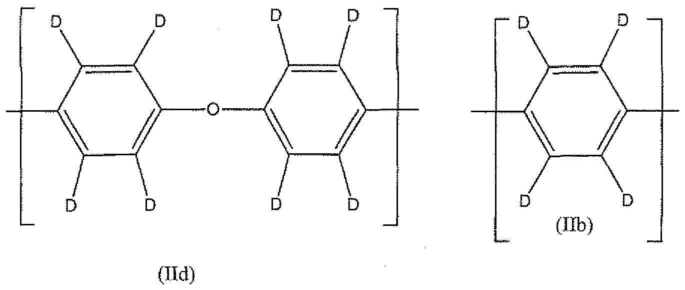
- polyimides comprising a repeat unit of following formula (Ic), a repeat unit of following formula (IIb) and a repeat unit of following formula (IId) :

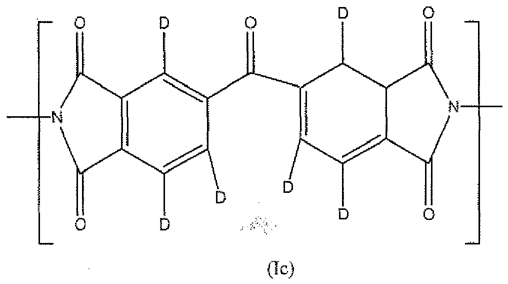

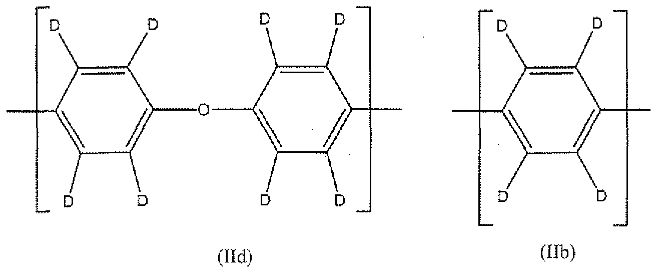

5. The deuterated polyimide as claimed in any one of claims 1 to 3, additionally comprising a unit corresponding to the following formula (III):

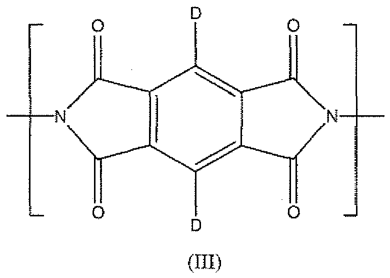

6. The deuterated polyimide as claimed in claim 5, comprising a repeat unit of following formula (Ia), a repeat unit of following formula (IIb) and a repeat unit of following formula (III):

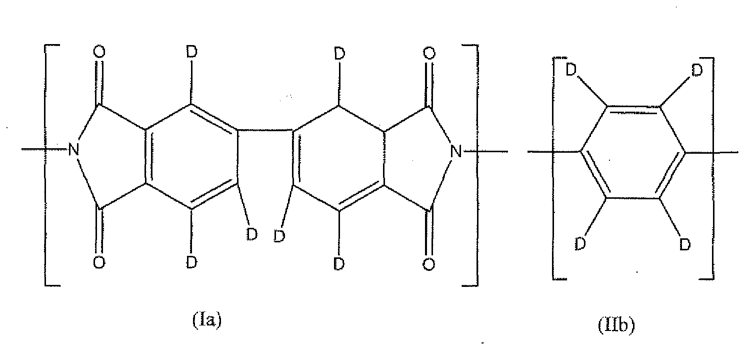

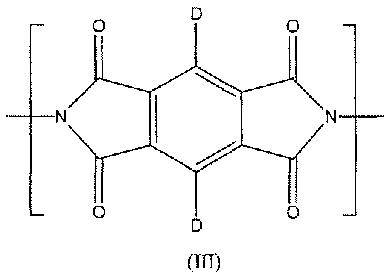

7. A process for the preparation of a deuterated polyimide as defined in any one of claims 1 to 3, said process comprising a stage consisting in treating, by heating at an appropriate temperature, a solution of a poly(amide-acid), the backbone of which comprises an alternation between at least one repeat unit of following formula (IV): 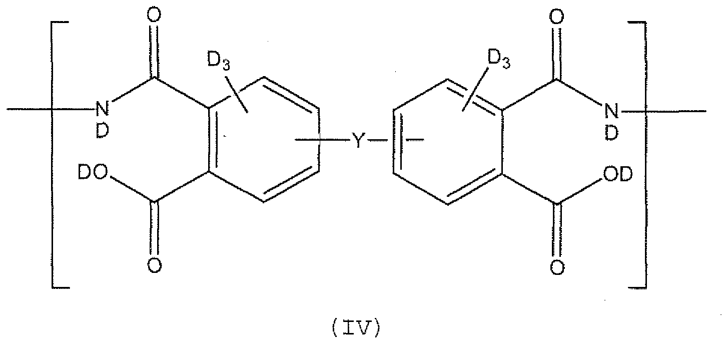 in which Y corresponds to the same definition as that given in claim 1; and
at least one repeat unit of formula (II) : 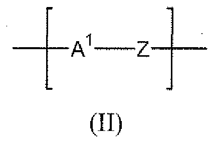 in which A¹ and Z correspond to the same definitions as those given in claim 1,
the appropriate heating temperature being determined so as to obtain complete imidization of said poly(amide-acid).

8. The preparation process as claimed in claim 7, in which the appropriate heating temperature is a temperature ranging from 80 to 400°C.

9. The preparation process as claimed in claim 7 or 8, in which the poly(amide-acid) solution is prepared by polycondensation, in a solvent, of at least one monomer of following formula (V): 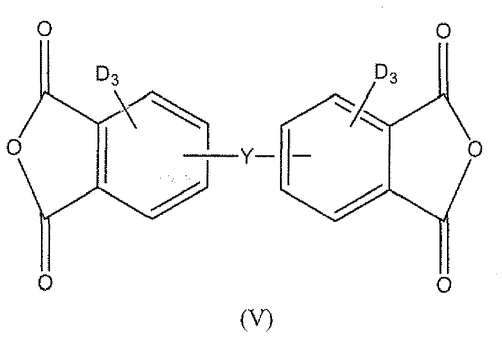 in which Y corresponds to the same definition as that given in claim 1, and
of at least one monomer of following formula (VI):
ND₂-A¹-Z-ND₂ (VI)
in which A¹ and Z correspond to the same definitions as those given in claim 1.

10. The preparation process as claimed in any one of claims 7 to 9, in which the solvent is a dipolar aprotic solvent chosen from the group consisting of N-methylpyrrolidone (NMP), dimethylformamide (DMF) and dimethylacetamide (DMAC).

11. A deuterated dianhydride monomer corresponding to the following formula (V): 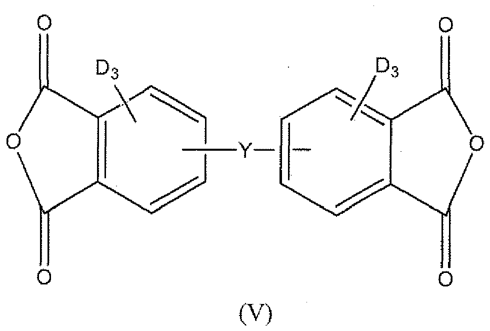 in which Y corresponds to the same definition as that given in claim 1.

12. The deuterated dianhydride monomers corresponding to one of the following formulae:

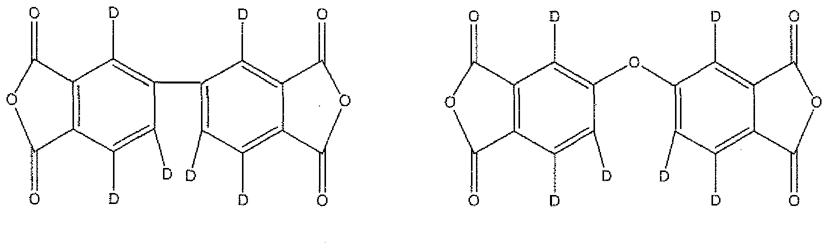

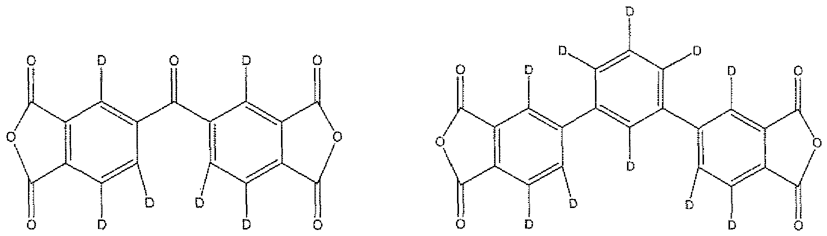

13. A process for the preparation of monomers of formula (V) : 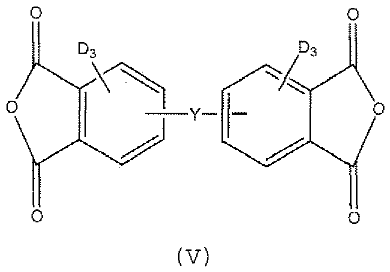 in which Y corresponds to the same definition as that given in claim 1, said process successively comprising the following stages:
- subjecting a compound of formula (VII): 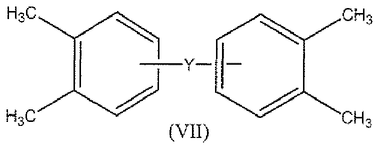 to deuteration, so as to obtain a compound of formula (VIII) : 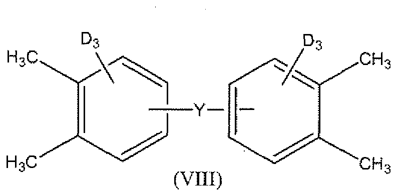
- subjecting the compound obtained above to oxidation, so as to obtain a compound of formula (IX): 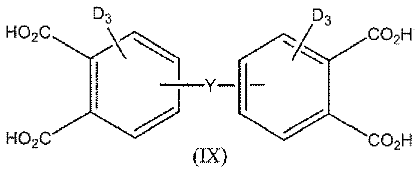
- subjecting the compound obtained above to cyclodehydration, so as to obtain the compound of formula (V).

14. A process for the preparation of deuterated diamine monomers of formula (VI): 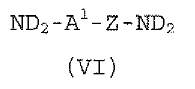 in which A¹ and Z correspond to the same definitions as those given in claim 1, said process successively comprising the following stages:
- reacting a compound of formula (XI) : 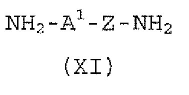 with an inorganic acid of formula HX, so as to obtain an ammonium salt of formula (XII): 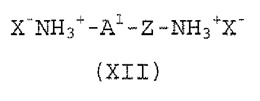 in which X represents a halide;
- reacting said ammonium salt with deuterated water under an appropriate pressure, followed by reacting with a base, so as to obtain the monomer of formula (VI).

15. A film based on a deuterated polyimide as defined in any one of claims 1 to 6.

16. The use of a deuterated polyimide as defined in any one of claims 1 to 6 as material which is transparent within the region from 2500 to 3500 cm⁻¹.

## Patentansprüche

1. Deuteriertes Polyimid, dessen Gerüst abwechseln umfasst:
- mindestens eine Wiederholungseinheit, die der folgenden Formel (I) entspricht: 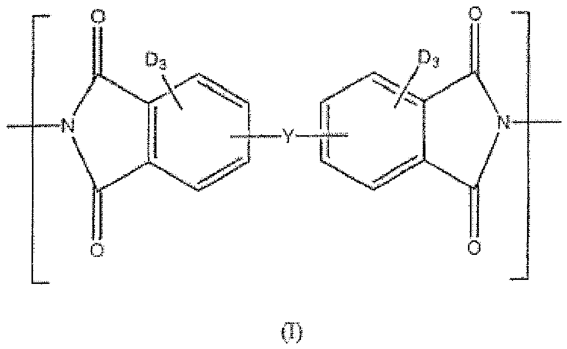 wobei
- Y eine Einfachbindung oder eine Spacergruppe darstellt; und
- mindestens eine Wiederholungseinheit, die der folgenden Formel (II) entspricht: 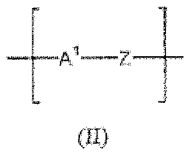 wobei:
- A¹ eine perdeuterierte aromatische Gruppe darstellt, die 6 bis 10 Kohlenstoffatome umfasst; und
- Z eine Einfachbindung oder eine Gruppe, ausgewählt aus -O-C₆D₄-, -CO-C₆D₄-, -C₆D₄-, darstellt.

2. Deuteriertes Polyimid gemäß Anspruch 1, wobei Y, wenn Y eine Spacergruppe ist, eine Gruppe, ausgewählt aus -O-, -CD₂-, -CO-, - SO₂-, -C₆D₄- ist.

3. Deuteriertes Polyimid gemäß Anspruch 1 oder 2, wobei die der Formel (II) entsprechende Wiederholungseinheit eine Wiederholungseinheit der folgenden Formel (IIa) ist: 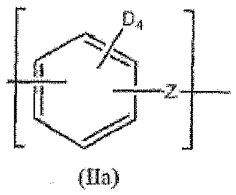 wobei Z der gleichen Definition, wie in Anspruch 1 gegeben, entspricht.

4. Deuteriertes Polyimid gemäß einem der Ansprüche 1 bis 3, ausgewählt aus der Gruppe, die aus den Polyimiden besteht, die ausgewählt sind aus:
- Polyimiden, die eine Einheit der folgenden Formel (Ia) und eine Wiederholungseinheit der folgenden Formel (IIb) umfassen: 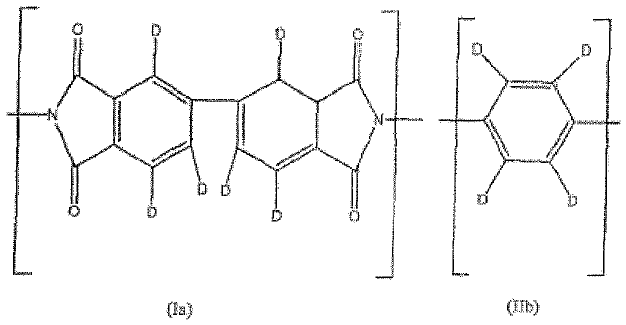
- Polyimiden, die eine Wiederholungseinheit der folgenden Formel (Ia) und eine Wiederholungseinheit der folgenden Formel (IIc) umfassen: 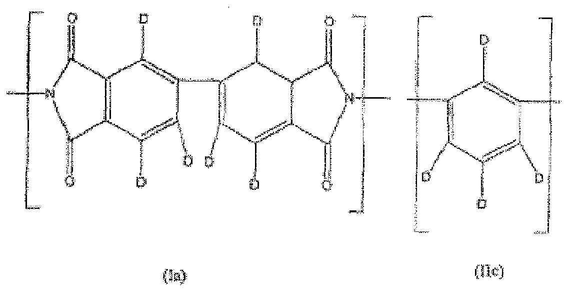
- Polyimiden, die eine Wiederholungseinheit der folgenden Formel (Ia) und eine Wiederholungseinheit der folgenden Formel (IId) umfassen: 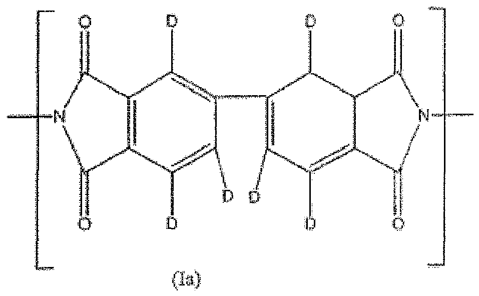 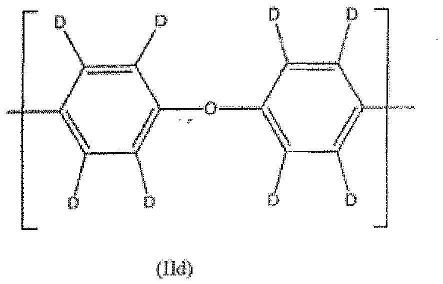
- Polyimiden, die eine Wiederholungseinheit der folgenden Formel (Ib) und eine Wiederholungseinheit der folgenden Formel (IIb) umfassen: 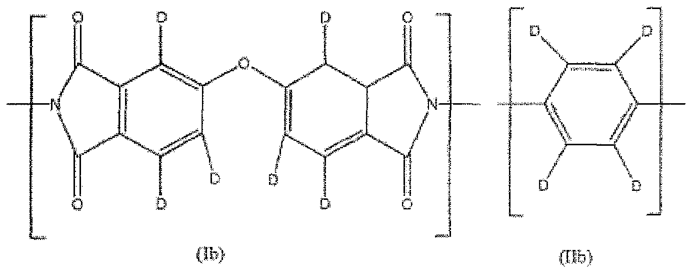
- Polyimiden, die eine Wiederholungseinheit der folgenden Formel (Ib) und eine Wiederholungseinheit der folgenden Formen (IId) umfassen: 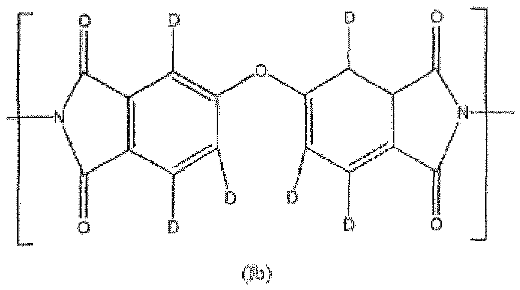 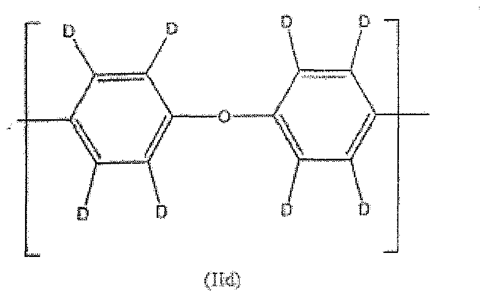
- Polyimiden, die eine Wiederholungseinheit der folgenden Formel (Ie) und eine Wiederholungseinheit der folgenden Formel (IIb) umfassen: 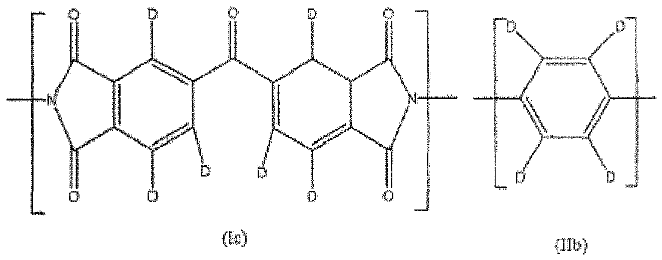
- Polyimiden, die eine Wiederholungseinheit der folgenden Formel (Ic) und eine Wiederholungseinheit der folgenden Formel (IId) umfassen: 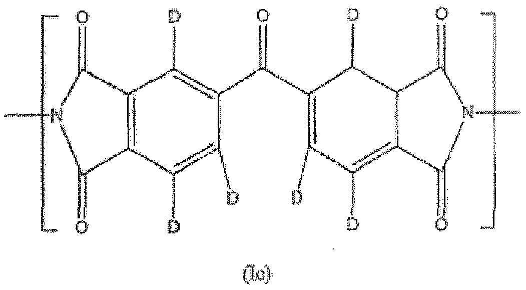 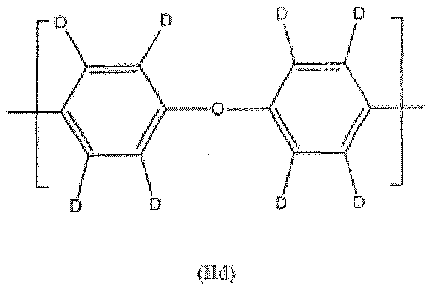
- Polyimiden, die eine Wiederholungseinheit der folgenden Formel (Id) und eine Wiederholungseinheit der folgenden Formel (IId) umfassen: 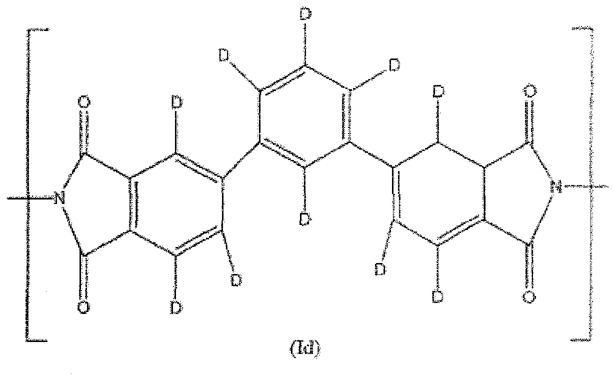 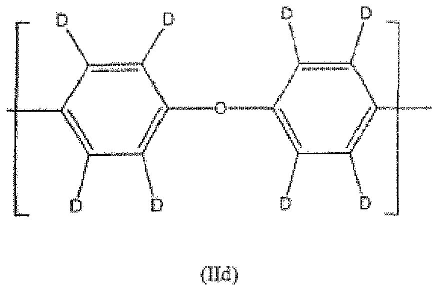
- Polyimiden, die eine Wiederholungseinheit der folgenden Formel (Ia), eine Wiederholungseinheit der folgenden Formel (IIb) und eine Wiederholungseinheit der folgenden Formel (IId) umfassen: 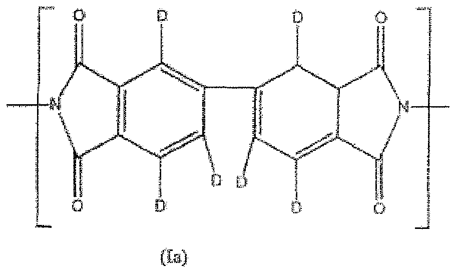 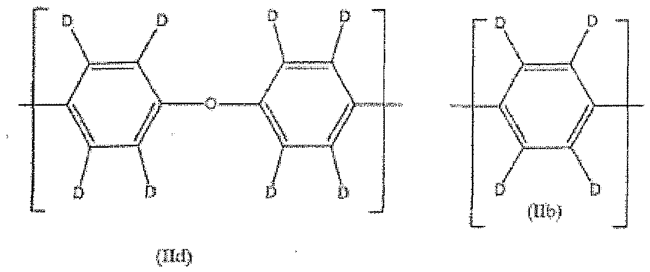
- Polyimide, die eine Wiederholungseinheit der folgenden Formel (Ic), eine Wiederholungseinheit der folgenden Formel (IIb) und eine Wiederholungseinheit der folgenden Formel (IId) umfassen:

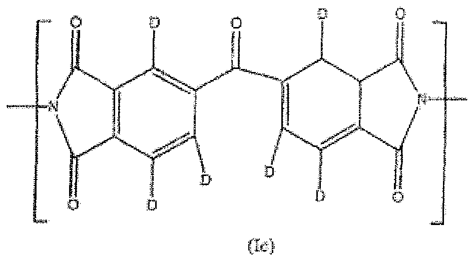

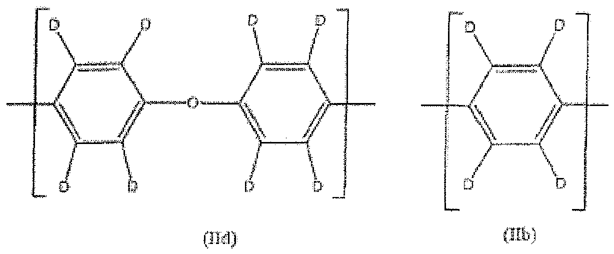

5. Deuteriertes Polyimid gemäß einem der Ansprüche 1 bis 3, welches weiterhin eine Wiederholungseinheit, die der folgenden Formel (III) entspricht, umfasst:

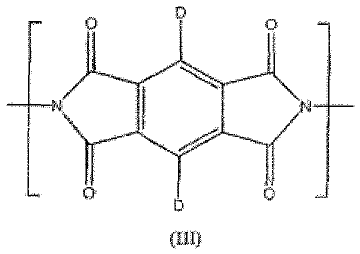

6. Deuteriertes Polyimide gemäß Anspruch 5, welches eine Wiederholungseinheit der folgenden Formel (la), eine Wiederholungseinheit der folgenden Formel (IIb) und eine Wiederholungseinheit der folgenden Formel (III) umfasst:

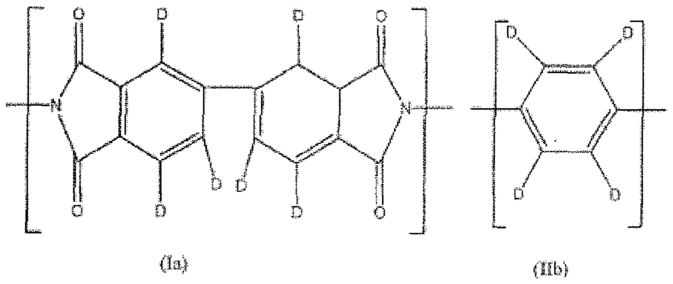

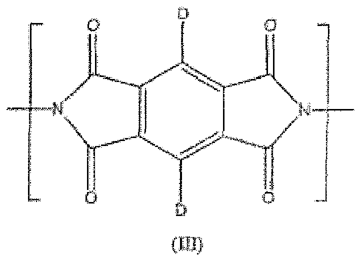

7. Verfahren zur Herstellung eines deuterierten Polyimids, wie gemäß einem der Ansprüche 1 bis 3 definiert, wobei das Verfahren einen Schritt umfasst, besteflend aus der Wärmebehandlung einer Lösung einer Poly(amidsäure), deren Gerüst abwechselnd mindestens eine Wiederholungseinheit der folgenden Formen (IV), 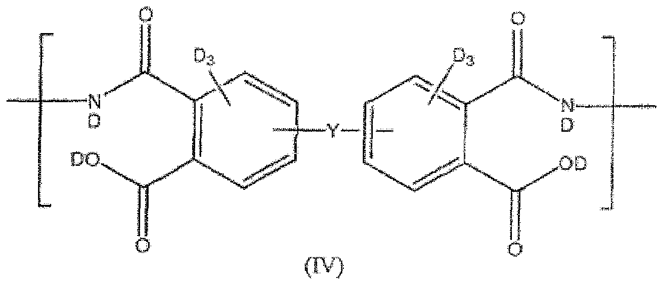 wobei Y der gleichen Definition, wie in Anspruch 1 gegeben, entspricht; und
mindestens eine Wiederholungseinheit der Formen (II) 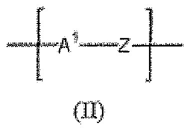 wobei A¹ und Z den gleichen Definitionen, wie in Anspruch 1 gegeben, entsprechen, umfasst, bei einer geeigneten Temperatur, wobei die geeignete Erwärmungstemperatur so bestimmt wird, dass eine vollständige Imidisierung der Poly(amidsäure) erreicht wird.

8. Herstellungsverfahren gemäß Anspruch 7, wobei die geeignete Erwärmungstemperatur eine Temperatur im Bereich von 80 bis 400°C ist.

9. Herstellungsverfahren gemäß Anspruch 7 oder 8, wobei die Poly(amidsäure)-Lösung durch Polykondensation von mindestens einem Monomer der folgenden Formel (V) 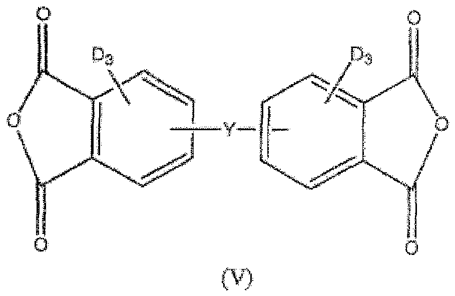 wobei Y der gleichen Definition, wie in Anspruch 1 gegeben, entspricht, und
mindestens einem Monomer der folgenden Formel (VI):
ND₂-A¹-Z-ND₂ (VI)
wobei A¹ und Z den gleichen Definitionen, wie in Anspruch 1 gegeben, entsprechen, in einem Lösungsmittel hergestellt wird.

10. Herstellungsverfahren gemäß einem der Anspruche 7 bis 9, wobei das Lösungsmittel ein dipolares aprotisches Lösungsmittel ist, das aus der Gruppe bestehend aus N-Methylpyrrolidon (NMP), Dimethylformamid (DMF) und Dimethylacetamid (DMAC) ausgewählt ist.

11. Deuteriertes Dianhydridmonomer, das der folgenden Formel (V) entspricht: 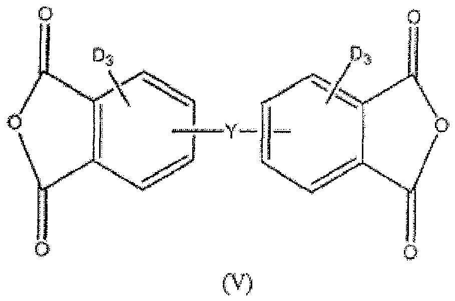 wobei Y der gleichen Definition, wie in Anspruch 1 gegeben, entspricht.

12. Deuterierte Dianhydridmonomere, die einer der folgenden Formeln entsprechen:

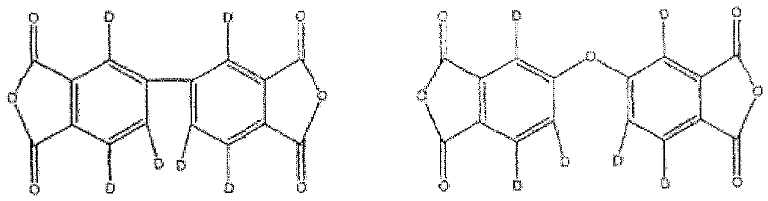

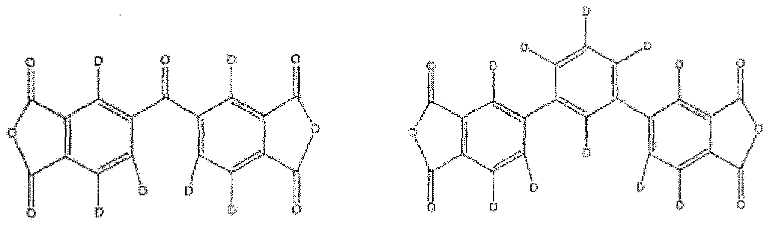

13. Verfahren zur Herstellung von Monomeren der Formel (V): 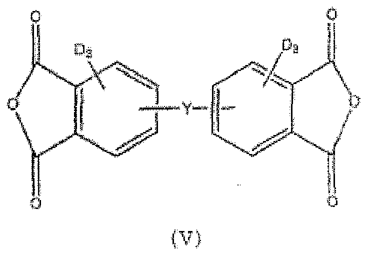 wobei Y der gleichen Definition, wie in Anspruch gegeben, entspricht, wobei das Verfahren nacheinander die folgenden Schritte umfasst:
- Unterziehen einer Verbindung der Formel (VII): 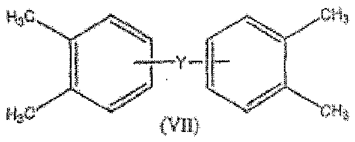 einer Deuterierung, um eine Verbindung der Formel (VIII) zu erhalten: 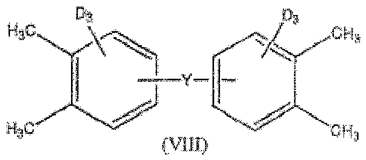
- Unterziehen der vorstehend erhaftenen Verbindung einer Oxidation, um eine Verbindung der Formel (IX) zu erhalten: 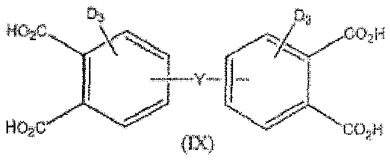
- Unterziehen der vorstehend erhaltenen Verbindung einer Cyclodehydratisierung, um die Verbindung der Formel (V) zu erhalten.

14. Verfahren zur Herstellung von deuterierten Diamin-Monomeren der Formel (VI): 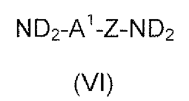 wobei A¹ und Z den gleichen Definitionen, wie in Anspruch 1 gegeben, entsprechen, wobei das Verfahren nacheinander die folgenden Schritte umfasst:
- Umsetzen einer Verbindung der Formel (XI): 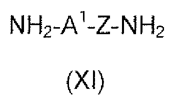 mit einer Mineralsäure der Formel HX, um ein Ammoniumsalz der Formel (XII) zu erhalten: 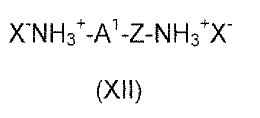 wobei X ein Halogenid darstellt;
- Umsetzen des Ammoniumsalzes mit deuteriertem Wasser bei einem geeignete Druck, gefolgt von einer Umsetzung mit einer Base, um das Monomer der Formel (VI) zu erhalten.

15. Folie auf der Basis eines deuterierten Polyimids, wie gemäß einem der Ansprüche 1 bis 6 definiert.

16. Verwerdung eines deuteherten Polyimid, wie gemäß einem der Ansprüche 1 bis 6 definiert, als transparentes Material im Bereich von 2500 bis 3500 cm⁻¹.
